# EUROPEAN PATENT SPECIFICATION

(11) **EP 1 392 334 B1**
(45) Date of publication and mention of the grant of the patent: **11.06.2008**
(21) Application number: 02711729.0
(22) Date of filing: 26.02.2002
(51) Int. Cl.: A61K 36/48, A61K 36/70, A61K 36/53, A61K 36/28, A61K 36/75, A61K 36/894, A61P 9/00, A61P 29/00, A61P 35/00

(54) **THERAPEUTIC METHODS USING HERBAL COMPOSITIONS**
THERAPEUTISCHE VERFAHREN UNTER VERWENDUNG VON PFLANZLICHEN ZUSAMMENSETZUNGEN
TRAITEMENTS BASES DE COMPOSITIONS PHYTOTHERAPIQUES

(30) Priority: 26.02.2001 US 793251
(43) Date of publication of application: 03.03.2004
(73) Proprietor: Global Cancer Strategies Ltd., Vancouver, British Columbia V6E 4M2 (CA)
(72) Inventor: TZE, John, Wah, Jun, deceased (CA); LIN,Peizhong, Cancer Inst., Chin.Acad.of Med.Sc., Beijing 100021 (CN); LAM, Stephen, Vancouver, British Columbia V6T 2E3 (CA); TAI, Joseph, North Vancouver, British Columbia V7L1T3 (CA)
(74) Representative: van Westenbrugge, Andries
(86) International application number: PCT/CA2002/000222
(87) International publication number: WO 2002/067961

(56) References cited:
- EP-A- 0 568 001
- EP-A- 0 665 017
- DATABASE EPODOC [Online] EUROPEAN PATENT OFFICE, THE HAGUE, NL "Method for preparation of anticancer medicine" XP002209477 & CN 1 047 975 A (LI CHENFU) 26 December 1990 (1990-12-26)
- DATABASE WPI Section Ch, Week 200146 Derwent Publications Ltd., London, GB; Class B04, AN 2001-426076 XP002209478 & CN 1 159 340 A (LI C), 17 September 1997 (1997-09-17)
- DATABASE WPI Section Ch, Week 199748 Derwent Publications Ltd., London, GB; Class B04, AN 1997-513921 XP002209479 & CN 1 125 591 A (LIU J), 3 July 1996 (1996-07-03)
- PATENT ABSTRACTS OF JAPAN vol. 1997, no. 05, 30 May 1997 (1997-05-30) & JP 09 012451 A (TERUMO CORP), 14 January 1997 (1997-01-14)
- DATABASE WPI Section Ch, Week 200212 Derwent Publications Ltd., London, GB; Class B04, AN 2002-083494 XP002209480 & CN 1 175 458 A (ZHANG Y), 11 March 1998 (1998-03-11)
- DATABASE WPI Section Ch, Week 200225 Derwent Publications Ltd., London, GB; Class B04, AN 2002-189049 XP002209481 & CN 1 324 657 A (MA C), 5 December 2001 (2001-12-05)
- DATABASE WPI Section Ch, Week 199747 Derwent Publications Ltd., London, GB; Class D11, AN 1997-504186 XP002209482 & CN 1 124 092 A (WANG Y), 12 June 1996 (1996-06-12)

## Description

The present invention relates to compositions comprising herbs derived from traditional Chinese medicine and their use as chemopreventive and therapeutic agents.

### BACKGROUND OF THE INVENTION

For the past twenty-five years there has been significant progress in the field of cancer research; however, in spite of these positive results, the mortality rate for the most common cancers still remains high. Indeed, the goal of the National Cancer Institute of a fifty percent reduction in overall cancer mortality by the year 2000 has not been met.

The term "cancer" is a general one referring to more than 100 forms of the disease which may manifest itself in almost every tissue type of the body. Of the myriad forms of cancer, lung cancer is the most common cause of death worldwide, followed by stomach cancer. Other common forms of cancer include cancers of the colon, rectum, breast, prostate, mouth and esophagus.

Lung cancer imposes an enormous burden on health care. The World Health Report 2000 estimates that lung cancer has resulted in 860,000 deaths among men and 333,000 deaths among women per year, and it is the leading cause of cancer deaths worldwide. In the United States and Canada, more people are dying from lung cancer than from breast cancer, colorectal cancer and prostate cancer combined. In addition, the incidence of lung cancer in women is rising at an average annual rate of 7% which is the most rapid rate of increase for any cancer. The most dominant cause of lung cancer is tobacco use, but occupational and environmental exposure to various other carcinogenic substances can also influence disease development. In long-term smokers, the risk of lung cancer never returns to the Abaseline@ level of a never-smoker, even years after smoking cessation. With a large reservoir (100 million in the United States and Canada alone) of current and former smokers, who are at risk, lung cancer will continue to be a major health problem for at least several more decades even if current efforts to curb tobacco smoking were successful. The overall five-year survival rate of lung cancer is less than 15%. Despite advances in modern medicine, the survival rate has not improved substantially over the last two decades. A different approach is therefore needed to control lung cancer.

Prostate cancer is the most commonly diagnosed male malignancy in the United States and the second leading cause of cancer death. It is estimated that in the year 2000, there will be 180,400 new cases and 31,900 deaths caused by prostate cancer. Although prostate cancer responds effectively to orchitectomy or antiandrogen therapy when detected at an early stage, over time, the residual androgen-insensitive cells recolonize, expand, and ultimately establish a hormone-resistant state that often results in fatality. It would be useful, therefore, to have a cancer treatment which could prevent the proliferation of prostate cancer and maintain it in a dormant state.

The incidence of gastric cancer has fallen in most countries but it is still the most common form of cancer in many countries of East Asia, including China. Globally, gastric cancer is the second most frequent cause of cancer death and it is estimated that in 1990, there were almost 800,000 new cases and about 630,000 deaths. Similarly, esophageal cancer, the eighth most common cancer worldwide and responsible for 316,000 new cases and 286,000 deaths in 1990, is also very common in China and other Asian countries. Both of these cancers, and especially esophageal cancer, have low survival rates and thus it would be beneficial to have an alternative treatment approach for these types of cancers.

Traditionally, the focus of cancer research has been in developing therapies and treatments for patients already afflicted with the disease. However, over the last few decades, new insights into the development of cancer as a disease have been gained. It is now understood that cancer is not the result of a single initiation event but of a gradual, multi-step process characterized by a period of several years between the initiation event and the onset of invasive or metastatic disease. In general, the process of carcinogenesis can be divided into three phases: initiation, promotion, and progression. In initiation, a fixed genetic mutation results from the interaction of a carcinogen with DNA. The extent of the molecular change depends on a number of factors including the nature of the carcinogen, the rate and type of carcinogenic metabolism and the response of the DNA repair systems. The next phase, promotion, may occur over extended periods of time and is characterized by the proliferation of the altered cells. This phase may be affected by agents that alter growth rates. During the final phase, progression, genetic and phenotypic changes occur which ultimately cause the development of premalignant lesions into invasive cancer.

The multi-step nature of carcinogenesis suggests the possibility of intervention at a precancerous state. This is the basis for chemoprevention, which refers to the use of natural or synthetic agents to prevent the development of cancer, either by blocking the DNA damage that initiates the carcinogenesis process or by arresting/regressing existing pre-malignant lesions.

Since the mid-1950s, research has been directed at finding compounds with potential chemopreventive properties. The search for these agents has demonstrated a unique challenge. Chemopreventive agents must have low toxicity and be relatively free of side effects because they are intended for administration to healthy people over long periods of time. This is in direct contrast to chemotherapy drugs, such as cis platinum or paclitaxel (Taxol^{™}), which are used as chemotherapeutic agents to treat people already afflicted with cancer. Chemotherapeutic agents are chosen for their ability to kill tumor cells but because they are also toxic to healthy cells, they usually cause harmful side effects.

One of the major sources of potential chemopreventive agents is plants. For example, consumption of cruciferous vegetables, such as broccoli, cauliflower and cabbage is associated with a lower risk of various cancers. Fruits and vegetables contain a number of potentially active chemopreventive compounds, such as carotenoids, dithiolthiones and isothiocyanates. They are capable of inhibiting the development of tumors of the lungs, colon, mammary glands and bladder in laboratory animals.

Three proof-of-principle clinical trials suggest that chemoprevention might be an effective strategy to control lung cancer. A study by Hong and co-workers in the United States showed that in patients with cured head and neck cancer, high dose 13-cis-retinoic acid for 12 months was more effective than placebo in preventing second primary cancers in the upper aerodigestive tract. However, 13-cis-retinoic acid at this dosage carries unacceptable toxicity for use in the general population. Another study by Pastorino and co-workers in Europe showed that retinol palmitate in a dose of 300,000 Units per day for 12 months was more effective than placebo in preventing second primary lung cancer. A third study in China found that daily doses of a combination of vitamins and minerals consisting of beta-carotene, vitamin E and selenium resulted in a 21% decrease in stomach cancer deaths in high-risk people in China. However, subsequent phase III clinical trials using beta-carotene or retinal (the Alpha-Tocopherol, Beta-Carotene Trial, the Beta-Carotene and Retinol Efficacy Trial, and the EUROSCAN study) failed to show a reduction in lung cancer incidence in high-risk individuals, such as heavy smokers with or without exposure to asbestos, compared to placebo. In fact, the use of beta-carotene in those who continued to smoke during the study was found to increase the risk of lung cancer. Several reasons were postulated to explain why chemopreventive treatment with retinoids was ineffective or even harmful in active smokers. There may be adverse interactions between tobacco carcinogens and the chemopreventive agent. Beta-carotene, for example, is a pro-oxidant at high arterial oxygen tension. It can enhance conversion of benzo[A]pyrene to the ultimate carcinogen as well as inducing cytochrome P450. Another reason for the lack of effect in active smokers is that ongoing tobacco carcinogen exposure may counteract the effect of the chemopreventive agent.

A number of chemopreventive agents are currently under clinical investigation. Examples of these include fenretinide, selenium, inhaled budesonide, COX-2 inhibitors, farnesyl transferase inhibitors, lipoxygenase inhibitors, EGF-kinase inhibitors and green tea. Although promising, it remains to be shown if these agents can be shown to be useful in ongoing clinical trials.

The best example to-date that chemoprevention can prevent cancer is Tamoxifen. Tamoxifen is an estrogen antagonist. In women at high risk of breast cancer, the Breast Cancer Prevention Trial showed a 49% decrease in invasive cancer and a 50% decrease in non-invasive breast cancer with Tamoxifen versus placebo (J Natl Cancer Inst 1998; 90:1371-88). In addition, there was a decrease in the incidence of fractures due to osteoporosis. However, there was a slight increase in the risk of endometrial cancer, deep venous thrombosis and pulmonary emboiism. More selective estrogen-receptor modulators such as Raloxifene, are being tested against Tamoxifen to determine if these agents may have similar chemopreventive effect but fewer side effects than Tamoxifen.

A variety of Chinese herbs have been used for centuries to treat different diseases. The great majority of these are empirical, open, non-randomized studies without placebo control groups. Although some of these herbs have been used to treat patients with cancer, they are not considered to be disease specific. Rather, they are used for Adispersing heat, detoxification, improving stasis and removing mass". Herbs such as *Sophora tonkinensis, Polygonum bistorta, Prunella vulgaris, Sonchus brachyotus, Dictamnus dasycarpus Turcz,* and *Dioscorea bulbifera* are known to have properties that may be useful for the prevention and treatment of cancers. One such composition is known as ZSP or Zeng Sheng Ping; however, the exact formulation of the composition is not known.

European Patent application 93 121109.8 describes a composition comprising *Sophora tonkinensis* Gapnep 42 mg, *Polygonum bistorta* L. 42 mg, *Prunella vulgaris* L. 42 mg *Sonchus brachyotus* DC 42 mg *Dictamnus dasycarpus Turcz* 21 mg, and *Dioscorea bulbifera* 10 mg which was useful for the treatment of patients with mouth, esophagus or digestive tract cancers.

It is well known that a chemopreventive agent that works in one organ may not work in the other organ. For example Tamoxifen, as discussed above, has been used in the treatment of breast cancer, but has not been found to be effective in any other cancers. Likewise, there is no evidence that other promising agents for preventing breast cancer, such as raloxifene and anastrozole, prevent other cancers. Therefore, persons would not predict that the composition described in the above referenced European Patent application, which was effective for treating esophagus cancer, would also show activity in other organs such as lung, prostate or breast. There is, thus, a need to determine other uses for this herbal composition and other related herbal compositions.

### SUMMARY OF THE INVENTION

Thus, according to the present invention there is provided a composition comprising herbs for uses in treating or preventing a variety of medical conditions.

Thus, according to this embodiment of the invention, there is provided the use of an effective amount of a composition comprising a mixture of *Sophora tonkinensis, Polygonum bistorta, Prunella vulgaris , Sonchus brachyotus, Dictamnus dasycarpus* and 0-1.4 wt.% *Dioscorea bulbifera* for treating a patient in need thereof, wherein the first five herbs are present in an amount from 5% to 33% and the sixth herb is present in an amount from 1.0% to 1.4% or wherein the first four herbs are present in an amount from 5% to 38% and the fifth herb is present in an amount from 2.5% to 19% and the sixth herb is present in an amount from 1.0% to 1.4%. In one example of this invention, there is provided a method of preventing or treating cancer, wherein said cancer is selected from the group consisting of lung cancer, prostate cancer, bladder cancer, breast cancer and leukaemia by administering to a patient in need thereof an effective amount of a composition as defined above.

In yet a further example of this embodiment of the invention, there is provided a method of treating or reducing the reoccurrence of cancer by administering to a patient in need thereof an effective amount of a composition as defined above.

In yet a further example of this embodiment of the invention, there is provided a method of treating or preventing cancer by administering to a patient in need thereof an effective amount of a composition as defined above, as adjuncts to conventional surgery, chemotherapy or radiotherapy treatments.
according to the present invention there is provided the use of an effective amount of a composition comprising a mixture of *Sophora tonkinensis, Polygonum bistorta, Prunella vulgaris , Sonchus brachyotus, Dictamnus dasycarpus* and *Dioscorea bulbifera* for treating a patient in need thereof
In one example of this embodiment of the invention, there is provided a method of treating *Helicobacter pylori* infection by administering to a patient in need thereof an effective amount of a composition as defined above.

In a further example of this embodiment of the invention, there is provided a method of treating or preventing a chronic inflammatory condition by administering to a patient in need thereof an effective amount of a composition as defined above.

In yet a further example of this embodiment of the invention, there is provided a method of treating or preventing cardiovascular disease by administering to a patient in need thereof an effective amount of a composition as defined above.

In yet a further example of this embodiment of the invention, there is provided a method of treating or preventing cerebral vascular disease by administering to a patient in need thereof an effective amount of a composition as defined above.

In a further example of this embodiment of the invention, there is provided a method of treating atrophic gastritis by administering to a patient in need thereof an effective amount of a composition as defined above.

According to the present invention, there is also provided a composition consisting essentially of (i) a mixture of about 5% to about 35% *Sophora tonkinensis,* about 5% to about 35% *Polygonum bistorta ,* about 5% to about 35% *Prunella vulgaris,* about 5% to about 35% *Sonchus brachyotus* and about 5% to about 35% *Dictamnus dasycarpus* or (ii) a mixture of about 6% to about 38% *Sophora tonkinensis,* about 6% to about 38% *Polygonum bistorta,* about 6% to about 38% *Prunella vulgaris,* about 6% to about 38% *Sonchus brachyotus* and about 3% to about 19% *Dictamnus dasycarpus* or (iii) a mixture of six herbs including about 5% to about 33% *Sophora tonkinensis,* about 5% to about 33% *Polygonum bistorta ,* about 5% to about 33% *Prunella vulgaris,* about 5% to about 33% *Sonchus brachyotus,* about 5% to about 33% *Dictamnus dasycarpus* and 1.0% to 1.4%up to 1.4 wt% *Dioscorea* bulbifera or (iiv) a mixture of six herbs including about 5% to about 38% *Sophora tonkinensis,* about 5% to about 38% *Polygonum bistorta ,* about 5% to about 38% *Prunella vulgaris,* about 5% to about 38% *Sonchus brachyotus,* about 2.5% to about 19% *Dictamnus dasycarpus* and 1.0 to 1.4%up to 1.4 wt% *Dioscorea bulbifera.*

### BRIEF DESCRIPTION OF THE DRAWINGS

These and other features of the invention will become more apparent from the following description in which reference is made to the appended drawings wherein:

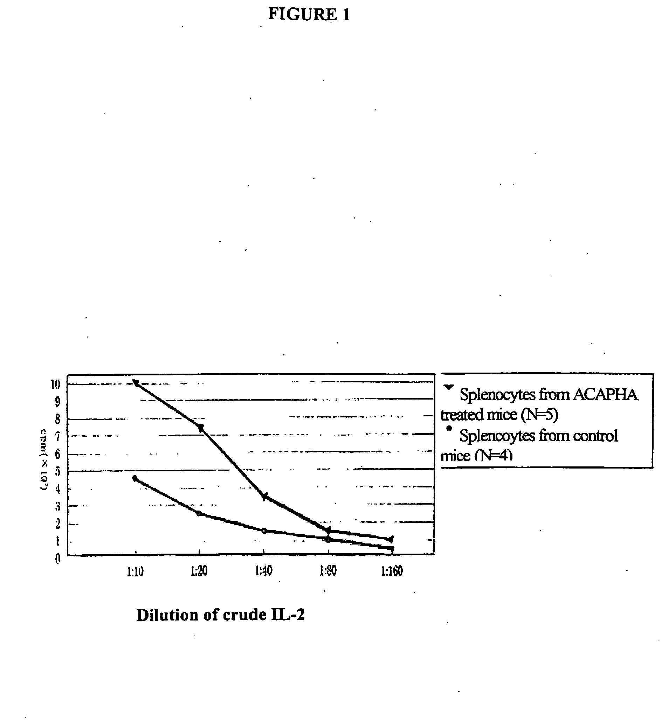

**FIGURE 1** shows the effect of ACAPHA on the activity of mouse spenocytes to produce IL-2.
**FIGURE 2** shows the effect of ACAPHA on the growth of S-180 tumor in mice after radiotherapy.
**FIGURE 3** is a bar graph showing the growth suppressive effect of 72 hours of treatment of 3 g/ml of ACAPHA 1 on androgen responsive LNCaP and androgen-refractory JCA-1, PC-3 and DU-145 prostate cancer cells.
**FIGURE 4** shows the effect of concentration (A, assessed after 72 hours of treatment) and duration of treatment (B) of ACAPHA 1 on androgen refractory JCA-1 prostate cancer cells in vitro.
**FIGURE 5** shows the comparison of effects of ACAPHA 1 (A1), ACAPHA 2 (A2), and ACAPHA 3 (A3) on colony formation using androgen-refractory (DU-145, JCA-1 and PC-3) and androgen-responsive (LNCaP) cells.
**FIGURE 6** compares the effects of 72 hours of treatment with various concentrations of ACAPHA 1 (A1), ACAPHA 2 (A2) and ACAPHA 3 (A3), respectively, on changes in the distribution of cell cycle phase progression in the androgen-refractory human prostate JCA-1 cell line.
**FIGURE 7** compares effects of 72 hours of treatment with various concentrations of ACAPHA 1 (A1), ACAPHA 2 (A2) and ACAPHA 3 (A3), respectively on changes in the distribution of cell cycle phase progression in the androgen-responsive human prostate LNCaP cells.
**FIGURE 8** shows the effects of various concentrations of ACAPHA 1 (A1), ACAPHA 2 (A2), and ACAPHA 3 (A3), respectively, on time-dependent increase in secreted PSA in media of androgen-responsive human prostate LNCaP cells. Cells were treated with ACAPHA for 1, 2, or 3 days. PSA in the culture media was determined using the TANDEM E kit from Beckman Coulter.
**FIGURE 9** shows the Western blot analysis of effects of various concentrations of ACAPHA 1 (A1), ACAPHA 2 (A2), and ACAPHA 3 (A3), respectively, on expression of secreted PSA in media of androgen-responsive human prostate LNCaP cells. Cells were treated with ACAPHA for 3 days. PSA in the culture media was determined using PSA cognate antibody and visualized by color reaction.
**FIGURE 10** shows the cell growth profiles of Jurkat and K562 leukemia cells treated with different concentrations of ACAPHA2 for 48 hours.
**FIGURE 11** shows the cell growth profiles of MCF7 and MDA-MG-468 breast cancer cells treated with different concentrations of ACAPHA2 for 96 hours.
**FIGURE 12** shows the cell growth profiles of Jurkat, K562, MCF7 and MDA-MB-468 cells treated with different concentrations of ACAPHA2.
**FIGURE 13** shows the cell cycle distribution of Jurkat leukemia cells after 48 hours of treatment with different concentrations of ACAPHA2.
**FIGURE 14** shows the cell cycle distribution of K562 leukemia cells after 48 hours of treatment with different concentrations of ACAPHA2.
**FIGURE 15** shows the cell cycle distribution of MCF7 breast cancer cells after 96 hours of treatment with different concentrations of ACAPHA2.
**FIGURE 16** shows the cell cycle distribution of MDA-MB-468 breast cancer cells after 96 hours of treatment with different concentrations of ACAPHA2.
**FIGURE 17** is a plot comparing the survival rates of patients treated with ACAPHA 1 as an adjunct therapy to radiotherapy compared to radiotherapy alone in patients with esophageal cancer.
**FIGURE 18** is a plot comparing the survival rates of patients treated with ACAPHA 1 as an adjunct therapy to surgery compared to surgery alone in patients with esophageal cancer.
**FIGURE 19** is a bar graph comparing the survival rates of patients treated with ACAPHA 1 as an adjunct to conventional surgery and radiotherapy 12, 24 and 36 months post-treatment.
**FIGURE 20** is a bar graph showing the clinical response of individuals having bronchial dysplasia to treatment with ACAPHA 1 and other putative chemopreventive agents six months post-treatment analyzed using site specific analysis. The bars represent complete regression of dysplasia (CR), no response (NR) and progression of disease (PD)/or appearance of new dysplastic lesions under treatment. Retinol=Vitamin A; Sialor=Anetholtrithione; Pulmicort= Inhaled Budesonide.
**FIGURE 21** is a bar graph of the data of Figure 20 analyzed using Person specific analysis.
**FIGURE 22** is a bar graph showing the clinical response of individuals having bronchial dysplasia to treatment with ACAPHA 1 six months post-treatment and six months off ACAPHA 1 analyzed using site specific analysis.
**FIGURE 23** is a bar graph of the data of Figure 22 analyzed using person specific analysis.

### DETAILED DESCRIPTION OF PREFERRED EMBODIMENT

The present invention relates to compositions comprising herbs and their use as chemopreventive and therapeutic agents.

In one embodiment of the present invention the composition comprises a mixture of *Sophora tonkinensis, Polygonum bistorta, Prunella vulgaris Sonchus brachyotus, Dictamnus dasycarpus,* and *Dioscorea bulbifera.*

In an embodiment of this invention, the first five herbs are present in an amount from about 5% to about 31 % and the sixth herb is present in an amount from about 1.0% to about 17.5%. In a further example of this embodiment, each of the first five herbs are present in an amount from about 10% to about 26%, and the sixth herb is present in an amount from about 5% to about 15%. In a further example of this embodiment, the first five herbs are each present in an amount from about 15% to about 21% and the sixth herb is present in an amount from about 7.5 to about 12.5%. In a further example of this embodiment, the first five herbs are present in an amount of about 18% and the sixth herb is present in an amount of about 10%.

In yet a further embodiment, the first five herbs are present in an amount from about 5% to about 33 % and the sixth herb is present in an amount from about 1.0% to about 1.4 %. In a further example of this embodiment, each of the first five herbs are present in an amount from about 10% to about 26%. In a further example of this embodiment, the first five herbs are each present in an amount from about 15% to about 21 %. In a further example of this embodiment, the first five herbs are present in an amount of about 18%

In yet a further embodiment the first four herbs are each present in an amount of about 5% to about 38%, the fifth herb is present in an amount of about 2.5% to about 19% and the sixth herb is present in an amount from about 1.0 to about 1.4 % One example of a composition in accordance with the present invention is a combination of *Sophora tonkinensis, Polygonum bistorta, Prunella vulgaris, Sonchus brachyotus, Dictamnus dasycarpus,* and *Dioscorea bulbifera* in an amount of about 21 %, 21 %, 21 %, 21 %, 10.5%, and 5.5%, respectively, and is referred to in the present application as ACAPHA 1 or ACAPHA - A1.

As will be shown in one of the examples which follow, some patients treated with a composition of ACAPHA 1 showed elevated liver enzymes. According to the present invention, a composition was provided wherein *Dioscorea bulbifera* was reduced by about 75%, which resulted in a corresponding reduction in high liver enzyme levels of patients being treated with this new composition. In one example of this embodiment there is provided a composition comprising a combination of *Sophora tonkinensis, Polygonum bistorta, Prunella vulgaris, Sonchus brachyotus, Dictamnus dasycarpus,* and *Dioscorea bulbifera* in an amount of about 21.9%, 21.9%, 21.9%, 21.9%, 11.0%, 1.4% and is referred to in the present application as ACAPHA 2 or ACAPHA - A2.

In another example of a composition not in accordance with the present invention *Dioscorea bulbifera* is eliminated entirely from the composition. Thus, according to this example, not in accordance with the invention, the composition comprises a combination of *Sophora tonkinensis, Polygonum bistorta, Prunella vulgaris, Sonchus brachyotus, Dictamnus dasycarpus,* and *Dioscorea bulbifera* in an amount of from 6%-38%, 6%-38%, 6%-38%, 6%-38%, and 3%-19%, respectively. In one example of this embodiment there is provided a composition comprising a combination of *Sophora tonkinensis, Polygonum bistorta, Prunella vulgaris, Sonchus brachyotus* and *Dictamnus dasycarpus* in an amount of about 22%, 22%, 22%, 22%, and 11%, respectively and is referred to in the present application as ACAPHA 3 or ACAPHA - A3.

According to the present invention, it was found that in clinical trials with ACAPHA 1 that certain percentage of patients liver enzymes, specifically AST (Aspartate Aminotransferase) was elevated. As a result, the ACAPHA 2 or 3 formulations, which have a lower concentration of *Dioscorea bulbifera,* as in ACAPHA 2 or a complete elimination of *Dioscorea bulbifera* from the composition, as in ACAPHA 3, are less toxic and thus are preferred over the ACAPHA 1 composition.

It is important to ensure that the herbs which are used according to the present invention are selected such that they contain only acceptable levels of contaminants such as metals or pesticides. Various regions of China have been surveyed and it was found that the component herbs may be harvested from the Guangxi, Hunan, Liaoning, Anhui, Hebei and Jiangsu regions of China during the summer and autumn months and no pesticides are used. The plants are purchased dried and whole or parts of these herbs are used in manufacturing.

The general manufacturing scheme is as follows: the herbs are either subjected to an aqueous extraction, the aqueous extract is then filtered if necessary to remove large particles, the aqueous extract is then dried to a powder. Alternatively, it is possible to use the herbs directly by grinding to a powder. The powdered herbs are then used in the production of the therapeutic in a variety of forms for administration.

Any suitable mode of delivery can be used according to the present invention. For example the composition of the present invention can be delivered orally by a pill, tablet, drink, candy or paste. The composition can also be delivered as a transdermal patch, by inhalation or suppository. Delivery of the composition as an injectable is also possible, according to the present invention. Therefore, the composition can be administered as a therapeutic agent or as a dietary supplement.

As previously noted, in one embodiment of the present invention, the tablets are formulated into 0.3g tablets. A typical daily dosage is about 1.2 to 6 g/day based on the body weight and/or severity of the condition. Generally the composition is given two to three times daily.

According to the present invention, the compositions are useful for the prevention and treatment of cancers. In one embodiment of the present invention, the cancers which are found to be treated or prevented are selected from the group consisting of lung cancer, liver cancer, bladder cancer, breast cancer, leukemia and prostate cancer.

In yet a further embodiment of the present invention, the compositions have been found to be useful as adjuncts to conventional surgery, chemotherapy or radiotherapy treatments in patients with cancer selected from but not limited to lung cancer, esophagus cancer, stomach cancer, oral cavity cancer, liver cancer, bladder cancer, breast cancer and prostate cancer and leukemia.

In the treatment of cancer, either alone or in combination with conventional cancer treatments including surgery, chemotherapy and radiotherapy, it has been found that the weight loss normally associated with cancer treatment did not occur in patients who were also treated with ACAPHA. An improvement in the quality of life therefore was found in the patients treated with ACAPHA. Furthermore, patients treated with ACAPHA showed a reduced level of cancer relapse or reoccurrence than patients who were not treated with ACAPHA.

Further, according to the present invention, the compositions are useful for the treatment or prevention of bronchial dysplasia. Bronchial dysplasia is a pre-malignant lesion of the bronchial epithelial. Currently, there is no standard treatment for bronchial dysplasia. Because tobacco use is on of the major causes of lung cancer, and former heavy smokers retain an elevated risk for lung cancer, even years after they have stopped smoking, the use of the compositions of the present invention for the treatment or prevention of bronchial dysplasia will have wide reaching benefits.

In a farther embodiment of the present invention, the compositions have been found to be useful for the treatment of *Helicobacter pylori* infection. Experimental results have shown broad immunostimulating activity of ACAPHA in enhancing cellular, hormonal and macrophage function. This activity would represent a potential mechanism for the anti-infective effect associated with ACAPHA in the treatment of *Helicobacter pylori* associated with atrophic gastritis as well as for the enhanced immune surveillance associated with ACAPHA effect in chemo prevention of tumor development of dysplasia. Thus, according to the present invention, the compositions have also been found useful for the treatment of atrophic gastritis.

In a further embodiment of the present invention, the composition has been found to be useful for preventing or treating a chronic inflammatory condition. Although not wishes to be bound by any particular theory, it is possible that the anti-anti-inflammatory activity is a result of ACAPHA' s anti oxidant capacity.

In yet a further embodiment of the present invention, the composition of the present invention has been found to be useful in the treatment or prevention of cardiovascular disease or cerebral vascular disease.

The present invention will now be illustrated by the following examples, which should not be considered limiting.

### EXAMPLES

### EXAMPLE 1: Antimutagenic and Anticancer-Promoting Properties

### A) Antimutagenic Properties

Ames tests demonstrated that ACAPHA1 has no mutagenic activity. ACAPHA 1 however, has antimutagenic activity. At 5, 25, 100 mg/plate, it inhibited the reverse mutation of TA100 induced by MNNG (N-methyl-N=-nitro-N=-nitrosoguanidine) at 0.5 µg/ptate (Table 1), AFB (Aflatoxin B) at 0.25 µg/plate (Table 2), Bap (Benzo(a)pyrene) at 0.5 µg/plate (Table 3), and that of TA98 induced by AFB at 0.25µg/plate (Table 4).

**Table 1: Inhibition of ACAPHA on reverse mutation of TA100 induced by MNNG**

| ACAPHA mg/plate mg/disc | S-9 | MNNG mg/disc | Colony X±SD | Inhibition rate % |
|---|---|---|---|---|
| 100 | C | C | 157±± 8.0 | |
| 0 | C | C | 146± 10.4 | |
| 0 | C | 0.5 | 298±19.6 | |
| 5 | C | 0.5 | 222±23.0 | 25.5 |
| 25 | C | 0.5 | 201±61.7 | 32.6 |
| 100 | c | 0.5 | 189±17.0 | 36.6 |

**Table 2: Inhibition of ACAPHA on reverse mutation of TA100 induced by AFB**

| ACAPHA mg/plate | S-9 | MNNG mg/disc | Colony X±SD | Inhibition rate % |
|---|---|---|---|---|
| 100 | -9 | C | 155± 16.5 | |
| 0 | 0 | C | 144±9.2 | |
| 0 | 0 | 0.25 | 1239±66.0 | |
| 5 | 0 | 0.25 | 1030±63.2 | 16.9 |
| 25 | 0 | 0.25 | 550±65.4 | 55.6 |
| 100 | 0 | 0.25 | 352±16.7 | 71.6 |

**Table 3: Inhibition of ACAPHA on reverse mutation of TA100 induced by BaP**

| ACAPHA mg/plate mg/d isc | S-9 | MNNG mg/disc | Colony X±SD | Inhibition rate % |
|---|---|---|---|---|
| 0 | -9 | C | 144± 9.2 | |
| 0 | 0 | 0.5 | 514±52.9 | |
| 5 | 0 | 0.5 | 465±86.4 | 10.0 |
| 25 | 0 | 0.5 | 254±15.2 | 50.6 |
| 100 | 0 | 0.5 | 258±19.6 | 49.8 |

**Table 4: Inhibition of ACAPHA on reverse mutation of TA98 induced by AFB**

| ACAPHA mg/plate mg/disc | S-9 | MNNG mg/disc | Colony X±SD | Inhibition rate % |
|---|---|---|---|---|
| 0 | C | C | 26±2.5 | |
| 0 | -9 | C | 48±3.1 | |
| 100 | C | C | 34±5.3 | |
| 100 | 0 | C | 46±8.3 | |
| 0 | 0 | 0.25 | 785±65.6 | |
| 5 | 0 | 0.25 | 628±83.6. | 20.0 |
| 25 | 0 | 0.25 | 468±41.2 | 40.4 |
| 100 | 0 | 0.25 | 69±8.1 | 91.2 |

### A) Antimicronucleus Properties

ACAPHA 1 inhibited micronucleus in mouse bone marrow induced by cyclophosphamide (CTX). The results in Tables 5 and 6 show that the quantity of micronucleus in polychromatic erythrocytes in the mouse bone marrow induced by CTX were significantly reduced by ACAPHA. The effects in 4 different dose groups were proportional to the dosage. The differences were very significant (p<0.001) comparing to the control CTX group. They also indicate that ACAPHA can protect the chromosomes from the injury.

**Table 5: Effects of ACAPHA on micronucleus rate of mouse bone marrow induced by CTX**

| Group | Dose(LD50) | Cells | Micronucleus Rate(%o) | P value |
|---|---|---|---|---|
| ACAPHA+CTX | 2 | 10000 | 6.9±0.4 | <0.001 |
| ACAPHA+CTX | 3 | 10000 | 9.1±0.6 | <0.001 |
| ACAPHA+CTX | 1/8 | 10000 | 9.4±0.5 | <0.001 |
| ACAPHA+CTX | 1/10 | 10000 | 10.3±0.4 | <0.001 |
| ACAPHA | 2 | 8000 | 1.4±0.2 | NS |
| NS | 0.5ml | 8000 | 2.0±0.3 | |
| CTX | 30mg/kg | 8000 | 17.3±1.1 | <0.00 1 |

**Table 6: Inhibitory effect of ACAPHA on the formation of micronucleus in bone marrow of mice induced by CTX.**

| Group | Dose(LD-50) | Inhibition rate(%) | Results |
|---|---|---|---|
| ACAPHA+CTX | 2 | 60.79 | 0 |
| ACAPHA+CTX | 3 | 48.29 | 0 |
| ACAPHA+CTX | 1/8 | 46.59 | 0 |
| ACAPHA+CTX | 1/10 | 40.47 | ± |

### A) Anti-promoting effect of ACAPHA

### i) The inhibitory effects of TPA

ACAPHA has significant inhibitory effects on the skin edema in the mouse ear (as shown in Table 7) and hyperplasia of the skin in the mouse back (as shown in Table 8). All the results indicate that ACAPHA has inhibitory effects on TPA (12-O-tetradecanoylphorbol-13-acetate), which means ACAPHA has the anti-promoting effect.

**Table 7: The inhibitory effect of ACAPHA on edema of mouse ear skin induced by TPA**

| TPA mg | ACAPHA mg | Weight of ear (mg) X±SE | P value |
|---|---|---|---|
| 0 | 0 | 6.36±0.22 | <0.001 |
| 0.4 | 0 | 13.24±0.56 | <0.001 |
| | | | |
| 0.4 | 0 | 16.22±0.59 | <0.02 |
| 0.4 | 2 | 9.87±1.37 | <0.02 |
| | | | |
| 0.4 | 0 | 17.56±1.51 | <0.01 |
| 0.4 | 4 | 10.48±0.23 | <0.01 |

**Table 8: The Inhibitory of ACAPHA on hyperplasia mouse back skin induced by TPA**

| TPA µg | ACAPHA Mg | Thickness of the skin X±SE | P |
|---|---|---|---|
| 0 | 0 | 20.00±1.69 | <0.001 |
| 1 | 0 | 42.29±2.37 | |
| | | | |
| 1 | 0 | 32.27±0.99 | <0.001 |
| 1 | 10 | 15.63±0.61 | |
| | | | |
| 1 | 0 | 39.17±1.67 | <0.001 |
| 1 | 10 | 20.00±1.29 | |

### ii The inhibition of ACAPHA 1 on the elevation of ODC activity in mouse epidermal cells induced by croton oil

Thirty mice were randomized into four groups, that is the control, croton oil, croton oil plus ACAPHA(5g/kg) and (10g/kg). The above doses were given at the day of and two days before the test of ODC (Ornithine decarboxylase) activity. The control and croton oil group received saline orally. According to O=Brien et al (O=Brien TG et al. Prog Clin Biol Res 1989; 298:213-13), ODC was taken from epithelial cells of the mice and its activity was tested. Table 9 shows that ACAPHA has significant inhibitory effects on the increase in ODC activity of mouse epidermal cells induced by croton oil. The effect was related to the dosage. It suggests that ACAPHA tablet has anti-promoting effects.

**Table 9: The Inhibitory effect of ACAPHA on the activity of ODC of epidermal cells of mice induced by croton oil**

| Group | ODC activity (pmol4CO2/30min/mg protein) |
|---|---|
| Control | 35.4±5.35 |
| Croton oil | 101.7±1.80 |
| Croton oil + ACAPHA 5g/kg | 82.5±10.56 |
| Croton oil + ACAPHA 10g/kg | 61.2±9.89 |

### EXAMPLE 2: The Anti-tumor and Tumor Prophylactic Effects of ACAPHA in Animals

### A) ACAPHA 1 and ACAPHA+5FU=s Inhibiting Effect on Induced Epithelial Carcinoma

BW Swiss female mice (22 to 28g) were selected for the experiment. N-Nitrososarcosine ethylester (2g/kg) were used to induce the dysplasia and carcinogenesis. Following the induction of dysplasia in the fore stomach, the carcinogen was discontinued. After 35 days ACAPHA and ACAPHA+5FU (5-fluorouracil) were then given at a dosage of 8 tablets/kg body weight, 3 times/wk, to a total of 20 times. The mice were weighed each week for the purpose of dose adjustment. After 11 weeks the mice were killed by cervical dislocation. After gross examination, the whole stomach was fixed in formalin and paraffin sections were stained with H&E (Hematoxylin & eosin). The lesions were classified into dysplasia, precancerous lesion, early stage and late stage of carcinoma. Table 10 shows that during the experiment, the weight growth of mice in ACAPHA group was similar to that of the control group. The dysplasia and carcinoma rates were significantly reduced by ACAPHA treatment, P<0.05. The dysplasia carcinoma rates were reduced even further when ACAPHA and 5FU were used together.

**Table 10: The inhibitory effect of ACAPHA and ACAPHA+SFU on epithelial carcinoma in the fore stomach of mice**

| **GROUP** | | **# of Animals** | **Pulmonary metastasis** | | **Inhibition rate** | **p** |
|---|---|---|---|---|---|---|
| | | | **Case** | **%** | **%** | |
| Control | Group 1 | 6 | 6 | 100.0 | | |
| Prevent | Group 2 | 6 | 5 | 83.3 | 17.7 | >0.05 |
| | Group 3 | 8 | 4 | 50.0 | 50 | <0.01 |

### B) The effect of ACAPHA 1 on induced dysplasia and canceration of hamster oral mucous membrane

Syria hamsters (43) provided by Beijing Biological Products Institute, ages were 4 to 8 weeks, 60 to 100 g were used. DMBA (7,12-dimethylbenz(a)anthracene)-acetone (0.5%) was rubbed in the middle of cheek pouch (close to the buccoanterior vein) of both sides of hamster three times per week for six weeks. Biopsies (13) were performed randomly after finishing the treatment course. About 50% of the hamster developed mild dysplasia in the oral cavity. ACAPHA was provided by Cancer Institute of Chinese Academy of Medical Sciences. Animals were divided to two groups: 17 animal (34 lesions) were fed ACAPHA 2 tablets per animal per day for 10 weeks. After that, the animals were monitored for 8 more weeks. Twenty-six animals (52 lesions) received no medications. Macroscopic observations include the appearing time, area, color, texture of the injury as well as the general condition. The tissues in the most conspicuous injuries of oral pouch were biopsied at 10^{th} week of medication and 8 weeks after stoppage of medication. The specimens were fixed in formalin and sections were stained with H&E. The results were classified into normal, simple hyperplasia, mild dysplasia, severe dysplasia and carcinoma based on Smith and Pindborg=s classification. The results were analyzed statistically. Changes in the mucous membrane of cheek pouch of hamster were detected after rubbing of DMBA for three weeks. Different leukoplakia could be seen with naked eyes. Plain leukoplakia comprised up to 65.3%, granular, 31.3%; pappilonodular, 3.2% and carcinoma, 0.5%. 13 biopsies were conducted randomly and the histology examination showed 7 with mild dysplasia(53.8%) and 6 with hyperplasia(46.2%). Changes in mucous membrane of cheek pouch of hamster after administration of ACAPHA for 10 weeks and after stoppage of ACAPHA for 8 weeks by microscope: After treatment for 10 weeks, the canceration rate in control group was 96.2% and treatment, 35.3%(Table 11). After fasting ACAPHA for 8 weeks, all the animals in control group became cancerous and the canceration rate in treatment group was 47.1 %(Table 12). There were a few cases that regressed to the normal mucous membrane after medication. They are 23.5% and 20.6% in each group. The cases that remained at dysplasia were 41.2% and 32.4% respectively in two groups.

**Table 11: Changes in mucous membrane of cheek pouch of hamster after administration of ACAPHA for 10 weeks**

| Group | No. of Cases | Normal | | Simple Hyperplasia | | Mild Dysplasia | | Severe Dysplasia | | Cancer | | Canceration |
|---|---|---|---|---|---|---|---|---|---|---|---|---|
| | | case | % | case | % | case | % | case | % | case | % | % |
| ACAPHA | 34 | 2 | 5.9 | 6 | 17.6 | 14 | 41.2 | 2 | 5.9 | 10 | 29.4 | 35.3 |
| Control | 52 | | | 1 | 1.9 | 1 | 1.9 | 3 | 5.8 | 47 | 90.4 | 96.2 |

**Table 12: Changes in mucous membrane of cheek pouch of hamster after stoppage of ACAPHA for 8 weeks**

| Group | No. of Cases | Normal | | Simple Hyperplasia | | Mild Dysplasia | | Severe Dysplasia | | Cancer | | Canceration |
|---|---|---|---|---|---|---|---|---|---|---|---|---|
| | | case | % | case | % | case | % | case | % | case | % | % |
| ACAPHA | 34 | 4 | 11.8 | 3 | 8.8 | 11 | 32.4 | 2 | 5.9 | 14 | 41.2 | 49.1 |
| Control | 52 | | | | | | | 52 | 100 | 52 | 100 | 100 |

The canceration rates (including cancer and severe dysplasia) and improvement rates (including hyperplasia and normal mucous membrane) were used as the indices of the efficacy with which statistical analyses were performed. There was very significant difference in the canceration rates between treatment and control groups (P<0.01). Similar results were observed after medication for 10 weeks and after stoppage for 8 weeks. There was no significant difference between two groups in the improvement rate. The results suggest that ACAPHA had some inhibitory effect on the canceration of precancerous lesions of oral mucous membrane. It could inhibit part of the canceration and the others remained at the stage of dysplasia with the possibility of canceration. It could prolong the stage of the precancerous stage. ACAPHA could not regress all the precancerous lesions to normal mucous membrane.

DMBA (5%) was rubbed on the oral mucous membrane of the hamsters three times per week for six weeks to induce dysplasia and then the hamsters were randomized into two groups. Forty-one hamsters in the control group received no further treatment. Sixteen hamsters received 2 crushed tablets of ACAPHA by gavage, three times per week for 12 weeks.

Macroscopic observations show that the mucous membrane in most parts of the dysplastic lesion (up to : of the total) changed from smooth to coarse then to smooth. Tumors with pedicle in the size of a rice grain were seen in minor part (1/4 of the total). In the control group, progressive changes were seen from smooth to coarse to nodular to tumor. Comparing with the animals treated with ACAPHA for 12 weeks, half of the dysplasia progressed to cancer. The cancer grew rapidly in size and the largest was up to I cubic centimeter. Table 13 shows the Microscopic observations after ACAPHA treatment for 12 weeks.

**Table 13: Microscopic observations after ACAPHA treated precancerous lesion of oral mucous membrane of hamster for 12 weeks**

| GROUP | No. of Cases | Normal | Hyperplasia | Mild dysplasia | Severe dysplasia | Carcinoma |
|---|---|---|---|---|---|---|
| Control | 20 | 0 | 3 | 4 | 6 | 7 |
| ACAPHA | 6 | 3 | - | | 2 | 1 |

Microscopic changes are more objective for diagnosis. Two microscopic criteria were used for judging the effect of ACAPHA on the development of dysplasia. The rate of canceration (including severe epidermal dysplasia) and the rate of improvement (including normal epithelium and epithelial hyperplasia) were analyzed with statistics. The U-test results are shown in Table 14.

**Table 14: The two rates after ACAPHA treated precancerous lesion of oral mucous membrane of hamster for 12 weeks**

| GROUP | Improvement rate | U test | Canceration Rate | U test |
|---|---|---|---|---|
| Control | 15% | P<0.05 | 65% | P>0.05 |
| ACAPHA | 50% | | 50% | |

There was significant difference in improvement rate between the ACAPHA treated and control group. It suggests that ACAPHA can reverse the canceration of dysplasia in oral mucous membrane of the hamster. No significant difference in canceration rate indicates that ACAPHA has no obvious effects on the canceration in the short term. Animals treated with ACAPHA for 12 weeks were reassessed macroscopically 3 months later. Of the 10 cases with dysplasia, 3 had tumors of 2-3mm³ in a limited area and the rest of them had a smooth mucous membrane. In the control group, all hamsters except two had a coarse mucous membrane with multiple and dispersed tumors. Most tumors were more than 1 cm³.

Animals treated with ACAPHA for 12 weeks were reassessed microscopically 3 months later. Tables 15 and 16 show that there was significant difference in improvement rates between the ACAPHA treatment and control groups (p<0.05).

**Table 15: Microscopic observations 3 month after ACAPHA treatment**

| GROUP | No of Cases | Normal | Hyperplasia | Mild dysplasia | Severe dysplasia | Carcinoma |
|---|---|---|---|---|---|---|
| Control | 21 | 0 | 6 | 2 | 1 | 12 |
| ACAPHA | 10 | 5 | 2 | 0 | 0 | 3 |

**Table 16: U-test for post-treatment observations**

| GROUP | Improvement rate | U | Canceration rate | U test |
|---|---|---|---|---|
| Control | 28.6% | P<0.05 | 61.9% | P<0.05 |
| ACAPHA | 70.0% | | 30.0% | |

The long-term observation verifies that ACAPHA has a positive effect on the regression of dysplasia of the oral muccous membrane of the hassters. Significant difference is seen in canceration rates too (p<0.05). It indicates that ACAPHA tablet has inhibitory effect on canceration on the dysplasia of the oral mucous membrane of the hamster.

Table 17 shows that measurements by multifunction morphometry shows that the nuclei in ACAPHA group are almost the same as those of the normal mucous membrane.

**Table 17: Nuclear measurements after administration of ACAPHA**

| GROUP | area(µm) | Circle(µm) | Maximum diameter (µm) | Morph Factor | Minimum Diameter (µm) |
|---|---|---|---|---|---|
| ACAPHA | 33.25±11.30 | 21.67±3.95 | 8.39±1.77 | 0.67±0.11 | 5.50±1.12 |
| Control | 40.40±17.23 | 23.95±5.30 | 9.17±2.16 | 0.60±0.14 | 6.13±1.42 |
| Normal | 32.29±12.19 | 22.15±3.91 | 8.61±1.49 | 0.49±0.15 | 5.55±1.33 |

Table 18 shows that there were significant morphological changes towards the normal values in the nuclei of dysplastic cells after ACAPHA treatment. The nuclei in the untreated control group show multimorphic and heteromorphic changes.

**Table 18: U-test for the measurement of nuclei**

| Item | ACAPHA vs Control | ACAPHA vs normal |
|---|---|---|
| Area | P<0.0 1 | P>0.05 |
| Circle | P<0.0 1 | P>0.05 |
| Max diameter | P<0.0 1 | P>0.05 |
| Morph Factor | P<0.01 | P<0.01 |
| Min Diameter | P<0.0 1 | P>0.05 |

The measurement of nuclear DNA in both groups is shown in Table 19.

**Table 19. Nuclear DNA content of mucous membrane cells of hamster oral cavity after ACAPHA treatment.**

| GROUP | DNA total | About 2C Cells(%) | 2.5C-5C Cells (%) | >5C cells (%) |
|---|---|---|---|---|
| Normal | 8.15±2.84 | 86,41 | 13,59 | 0 |
| ACAPHA treatment | 9.91±3.54 | 68,03 | 31,71 | 0,26 |
| Control no treatment | 12.50±5.61 | 45,94 | 48,69 | 5,38 |

U-test for the results in Table 19 is shown in Table 20.

**Table 20. U-test for nuclear DNA.**

| Item | ACAPHA vs Control | ACAPHA vs Normal |
|---|---|---|
| DNA total | P<0.01 | P<0.01 |
| 2C cells(%) | P<0.01 | P<0.01 |
| 2.5C-5C cells(%) | P<0.0 | P<0.01 |
| >5C cells(%) | P<0.0 1 | ----- |

The main peak in the bar chart of the DNA in ACAPHA group is 2C cells group, which is similar to the normal. The main peak in the control untreated group is 2.5C-4.5C multiples. There are very significant differences in the DNA quantity and multiples (>5C) between two groups.(p<0.01). The above results suggested that ACAPHA has obvious short term and long-term inhibitory effects on the development of dysplasia and canceration of the oral mucous membrane of the hamster induced by DMBA. The epithelium after treatment has significant differences in both morphology and nuclear DNA content from the dysplasia tissue and is similar to the normal tissue.

### EXAMPLE 3: The Effect of ACAPHA 1 on Carcinoma of Nasopharynx in Rats Induced with Nitrosamine DNP (N N=-dinitrosopiperazine)

DNP (0.5%) was injected SC in the axilla of each rat twice a week for seven weeks to induce nasopharyngeal carcinoma. The Prevention Group began to receive ACAPHA one day before the DNP and treatment group began to receive ACAPHA one day after fourteen DNP injections. Table 21 shows that the canceration rate in the control group was 60%, significantly higher than the prevention group of 36.5% (p<0.05). The results indicated that ACAPHA has preventive effect on the development of nasopharyngeal carcinoma. ACAPHA treatment also has inhibitory effect. The data show that ACAPHA significantly reduced the development of precancerous lesions and canceration rates (P<0.01).

**Table 21: The prevention and the treatment with ACAPHA on precancerous lesion of nasopharynx in rats induced by DNP**

| GROUP | Number of feeding | N | Precancerous Lesions | | | | Canceration % | P |
|---|---|---|---|---|---|---|---|---|
| | | | I | II | III | IV | | |
| Control | - | 60 | 60 | 57 | 23 | 13 | 36(60.0) | |
| Prevention | 60 | 52 | 52 | 50 | 8 | 11 | 19(36.5) | <0.05 |
| Treatment | 5 | 29 | 29 | 25 | 1 | 6 | 1(24.1) | <0.01 |

### EXAMPLE 4: The Inhibitory Effect of ACAPHA 1 on Dysplasia of Bladder Mucous Membranes of Rats Iduced by Nitrosamine BBN (N-butyl-N-(4-hydroxybutyl)nitrosamine)

BBN was given three times a week for 24 weeks to induce bladder mucous dysplasia in rats. Animals in the treatment group received 4 ACAPHA tablets orally, three times per week until the end of the experiment. The control animals did not receive any treatment. The bladder wall of the animals in the control group thickened following BBN treatment. Tumors were seen in all bladders in this group. The bladder was filled with tumor nodules in some animals. The diameter of the tumors ranged from 0.5cm to 2cm, some with central necrosis. In the ACAPHA treatment group, the bladder mucous membrane was only slightly thickened. Large tumor nodules were only seen in a few rats. Most of them had small tumors or had no tumors. There was papilloma in parts of the bladder wall of animals in all groups. The proliferation of epithelium was less than six layers and lined with papilla. Differentiation of the cells was good and little mitosis could be seen. Carcinomas were seen too in part of the animals in all groups. The epithelium proliferation in the carcinoma was more than six layers. The nucleus fission was seen frequently. The papilla was not clear or disappeared. The carcinoma cells disperse between the normal cells like a nest.

**Table 22: The inhibitory effect of ACAPHA on canceration of dysplasia rat rats bladder induced by BBN**

| GROUP | Animal | Papilloma | | Cystocarcinoma | | Inhibition rate |
|---|---|---|---|---|---|---|
| | | Case | % | Case | % | % |
| Control | 20 | 20 | 100.0 | 12 | 60.0 | |
| ACAPHA | 18 | 12 | 66.7* | 1 | 5.6* | 90.7 |

| | | | | | | |
|---|---|---|---|---|---|---|
| * P<0.05 * P<0.01 | | | | | | |

Papilloma was found in all 20 rats of the control group. Carcinoma was seen in the bladders of 12 rats (60%). Twelve of the eighteen rats in the ACAPHA groups had papilloma (66.7%) and only one had carcinoma.(5.6%). It suggests very significant difference from the control group(p<0.01). The inhibitory rate was up to 90% (Table 22). The results suggest that ACAPHA has significant inhibitory effects on the canceration of the epithelium dysplasia of the rat bladder induced by the BBN.

### EXAMPLE 5: The Effect of ACAPHA 1 on Lung Metastasis in Mouse Tumor Model

DBA/2 mouse no.2 spontaneous lung cancer was transplanted to the mouse SC. The latent period was 5 to 8 days and the metastatic rate was 100%. The lung metastatic carcinoma was a transparent node. The survival time of mouse with the tumor was around 35 days. Mice were divided into three groups: the control group with 6 mice. The second group: 6 mice fed with ACAPHA twice before the transplant and 8 doses after. The third group 8 mice treated with 9 doses of ACAPHA before the transplant and 8 doses after. The survival rate of the transplant in both the control and the prevention group was 100%. It was easier to remove the metastatic carcinoma in the prevention group than in the control group. The metastatic carcinoma was bigger and adhered closely to the skin in the control group. Results in Table 23 shows that ACAPHA had significant inhibitory effects on metastatic carcinoma.

**Table 23: Inhibitory effect of ACAPHA on lung metastasis**

| GROUP | | | # of Animals | Average weight of tumor (g) | Min-Max (g) | Inhibition rate(%) | p |
|---|---|---|---|---|---|---|---|
| Control | Group 1 | | 6 | 3.04 | 2.35-3.50 | | |
| Prevention | | Group 2 | 6 | 2.17 | 1.48-2.78 | 28,6 | <0.01 |
| | | Group 3 | 8 | 1.55 | 0.65-2.85 | 49 | <0.01 |
| | | Total | 14 | 1.81 | 0.65-2.85 | 40,5 | <0.01 |

Macroscopic metastatic carcinoma was seen in the lungs of all 6 mice in the control group, but only 3 in the second group and 1 in the third group. The inhibition rates were 50% and 87.5% respectively.(Table 24).

**Table 24: The inhibitory effect of ACAPHA on Lung metastasis of mice(1)**

| GROUP | | # of Animals | Lung metastasis | | Inhibition rate (%) | P |
|---|---|---|---|---|---|---|
| | | | Cases | % | | |
| Control | Group (1) | 6 | 6 | 100.0 | | |
| Prevent | Group (2) | 6 | 3 | 50.0 | 50.0 | <0.01 |
| | Group (3) | 8 | 1 | 12.5 | 87.5 | <0.01 |
| | Total | 14 | 4 | 28.6 | 71.4 | <0,01 |

There were differences in the number and the size of metastatic carcinoma between the control group and the prevention group. In the lungs of all 6 mice in the control group, there were dispersing transparent metastatic tumor nodules of the size of millet were dispersed throughout the lung. In the prevention groups, the numbers and size were smaller than those in the control group. Of the 3 mice with metastasis in the group 2, one mouse only had 2 nodules the size of a pinhead. In the lung of the only mouse in group 3, 5 pinhead-sized nodules were seen. The average of the weight of lung over body weight of mice in the control group was 0.0143 and the average of 4 mice in the two treatment groups was 0.011. The difference was 23.1 %.(Table 25).

**Table 25: The inhibitory effect of ACAPHA on Lung metastasis in mice(2)**

| GROUP | # of Animals | Metastasis | Lung +tumor weight /body weight | Inhibition (%) |
|---|---|---|---|---|
| Control (1) | 6 | 6 | 0.0143 | |
| Prevent (2+3) | 14 | 4 | 0.0110 | 23.1 |

The pathological results are shown in Table 26. Lung metastasis was seen in all 6 mice in control group, and 5 of 6 in group 2 mice treated with ACAPHA. Two of these were detected microscopically. Of the 8 mice in group 3 treated with ACAPHA, 4 mice has lung metastasis but the rate was 50%, significantly lower than the control group. 3 of these four were detected microscopically.

**Table 26: The inhibitory effects of ACAPHA on microscopic pulmonary metastasis in mice**

| GROUP | | # of Animals | Pulmonary metastasis | | Inhibition rate | p |
|---|---|---|---|---|---|---|
| | | | Case | % | % | |
| Control | Group 1 | 6 | 6 | 100.0 | | |
| Prevent | Group 2 | 6 | 5 | 83.3 | 17.7 | >0.05 |
| | Group 3 | 8 | 4 | 50.0 | 50 | <0.01 |

The above results suggest that ACAPHA has significant inhibitory effect on lung metastasis and the longer ACAPHA was fed before the transplant, the more effective the treatment.

### EXAMPLE 6: Effect of ACAPHA 1 on mice exposed to lethal dose of X-Ray.

Mice in the treatment group received ACAPHA for 7 days (3g/kgX14) before and after the exposure. Control animals received 0.4ml tap water orally. X-ray was originated from a Phillips linear accelerator at 8MEV/20X20cm/100cm/250GY. The LD50 of total body irradiation in mouse was 400- 600 GY. 700GY X-ray was used for the test and the survival rate in 30 days was recorded.

**Table 27. Effect of ACAPHA on mice exposed to lethal dose of X-ray**

| No | Group | N | Weight(g) | ACAPHA administration | x-ray (GY) | 30Day survival | Survival rate | P value |
|---|---|---|---|---|---|---|---|---|
| | | | X±SD | | | | (%) | |
| 1 | Control | 10 | 23.75±0.93 | Water0.4ml/each P. 0×14 | 700 | 1 | 10 | |
| | ACAPHA | 10 | 23.45±0.9 1 | 3g/kgP.0×14 | 700 | 6 | 60 | <0.02 |
| 2 | Control | 10 | 19.80±1.75 | Water0.4ml/each P. 0×14 | 750 | 0 | 0 | |
| | ACAPHA | 10 | 19.70±1.85 | 3g/kgP.0×14 | 750 | 3 | 30 | <0.05 |

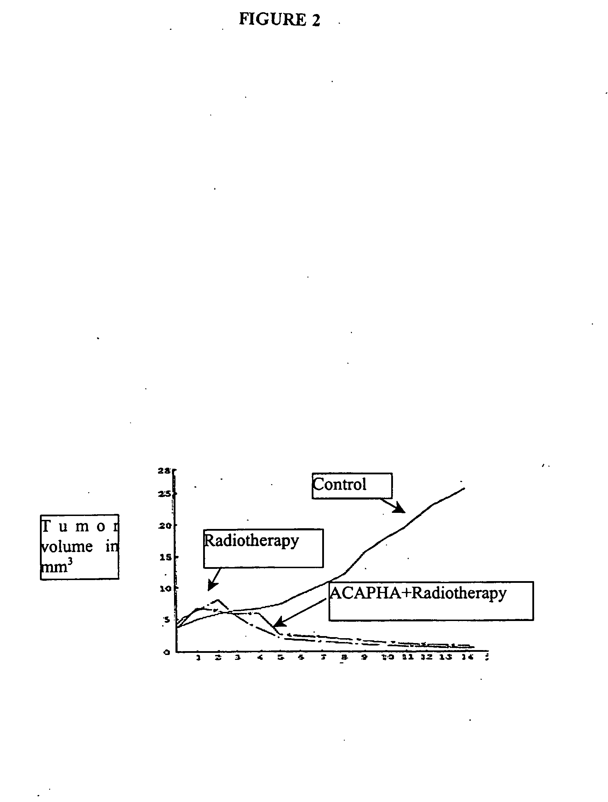

Table 27 shows that at 700GY exposure, the survival rate in ACAPHA group was 60%, significantly improved over the 10% in the control group (p<0.02). At exposure dose of 750GY, the survival rate of ACAPHA group was 30% and the control 0 (p<0.05). To further test the effects of ACAPHA on the sensitivity of tumors to X-ray exposure, 30 S 180 mice were randomly into 3 groups. The control group received no treatment after the tumor transplant. For the exposure only group, mice were exposed to X-ray at 2400GY locally once when the tumor grew to 4mm³. The same dose was given to mice in the third group, but 3g/kg of ACAPHA was fed for 10 days starting on the same day of the exposure. Figure 2 shows that tumors in the control group grew well while the curves for the other two groups were low and parallel. The results indicate that ACAPHA has no effects on the sensitivity of the tumor to X-ray.

### EXAMPLE 7: Effect of ACAPHA on Human Prostate Cancer Cell Growth

Prostate cancer is an increasing public health problem among men in the United States. If caught at an early stage, prostate cancer often responds to orchitectomy or antiandrogen therapy. However, over time residual androgen-insensitive cells will recolonize, expand and establish a hormone-resistant state, leading to a recurrence of the prostate cancer. In the following example the effect of ACAPHA on androgen responsive and androgen refractory human prostate cell lines was investigated. The results showed that ACAPHA is an effective inhibitor of human prostate cell growth.

Human prostate cell lines (DU-145, LNCaP, and PC-3) were obtained from American Type Culture Collection (Rockville, MD). These cell lines were derived from various sites to which primary tumor had metastasized. The JCA-1 cells were established at the New York Medical College from the primary prostate cancer site prior to the administration of hormone. Androgen-responsive LNCaP cells may be representative of latent prostate cancer, whereas androgen-refractory JCA-1, PC-3, and DU-145 cells are considered to better represent advanced prostate cancer. Cells were maintained in RPMI 1640 media containing 2 mM glutamine, 100 units/ml penicillin, 100 g/ml streptomycin, and supplemented with 10% heat-inactivated fetal calf serum.

ACAPHA solutions for the *in vitro* studies were prepared by stirring ACAPHA powder or tablets in 70% ethanol (340 mg/ml) at 150 rpm for one hour at room temperature. Insoluble material was removed by centrifugation. The ethanolic extracts were sterilized by passing through 0.22 m filters and kept in aliquots in a refrigerator. Final concentrations of ACAPHA used for different experiments were prepared by diluting the stock with RPMI1640. Control cultures received the carrier solvent. Two different lots of ACAPHA 1 tablets and three different formulations of ACAPHA in powder form ACAPHA 1, ACAPHA 2 and ACAPHA 3 were prepared in an identical manner.

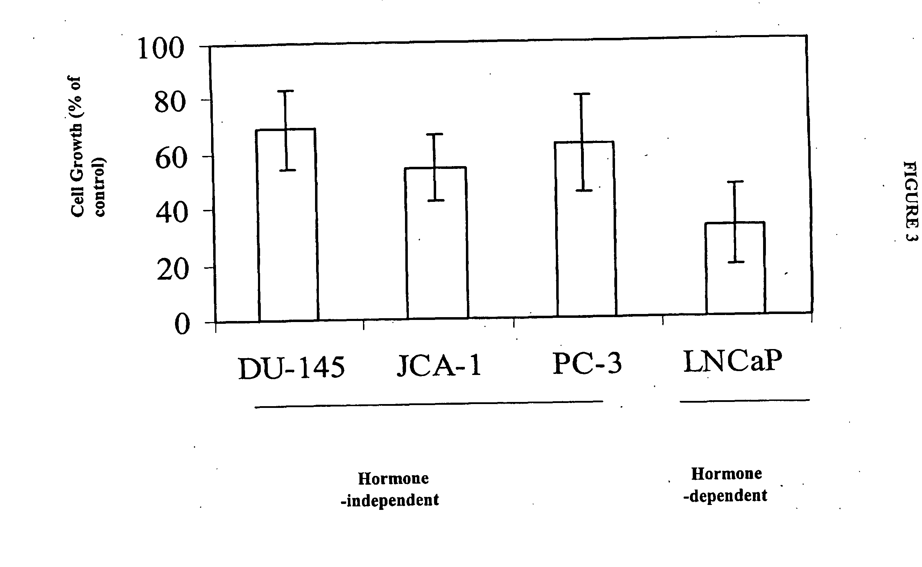

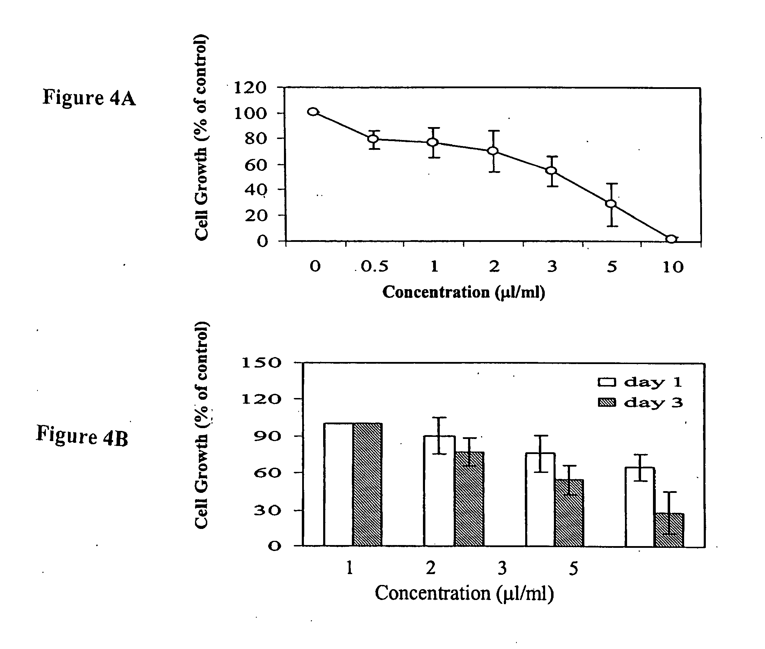

Effect of ACAPHA on Growth of Prostate Cancer Cells. Prostate cancer cell lines were treated with different concentrations of ACAPHA and after 24, 48 and 72 hours, cells were harvested. Cell number was determined by hemocytometer and cell viability was assessed by trypan dye exclusion assay. Ethanolic extracts of ACAPHA 1 inhibited cell proliferation in both hormone-dependent and hormone-independent prostate cancer cells. At 3 g/ml of ACAPHA 1, hormone-dependent LNCaP cells was more sensitive to the treatment than hormone-refractory cell lines. The growth suppression is shown as follows: LNCaP (67.4%)>JCA-1 (45.7%) > PC-3 (37.5%) > DU-145 (31.1%) (Figure 3). Further studies showed that growth suppression of JCA-1 cells was both concentration and time dependent. This cell line has been shown to be refractory to inhibition by resveratrol and other agents currently used for treatment of prostate cancer. With 3 g/ml of ACAPHA 1 treatment it reached its IC₅₀, and dramatic growth inhibition accompanying with decreasing in cell viability was found at 5 and 10 g/ml of ACAPHA 1 (Figure 4A) and more significant effect was found in day 3 of treatment (Figure 4B). The study demonstrates that ACAPHA 1 has potent inhibitory activity on both androgen-responsive and androgen-refractory human prostate cells. Therefore, ACAPHA 1 may be useful for patients who have failed to respond to conventional therapy.

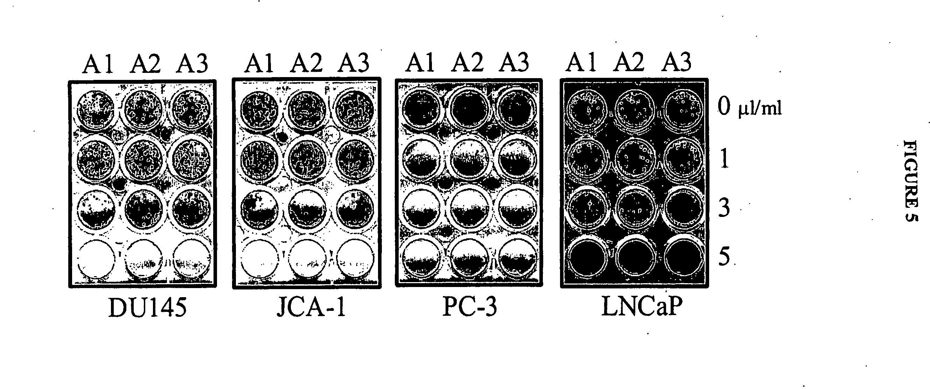

Effect of ACAPHA on Clonogenicity of Prostate Cancer Cells. The growth suppressive effect of three different formulations (compositions) of ACAPHA was tested with the clonogenic assay. Figure 5 shows that dose-dependent inhibition was observed whereas formulation variation was not seen. ACAPHA 1, 2 and 3 effectively suppressed colony formation against all four human prostate cancer cell lines.

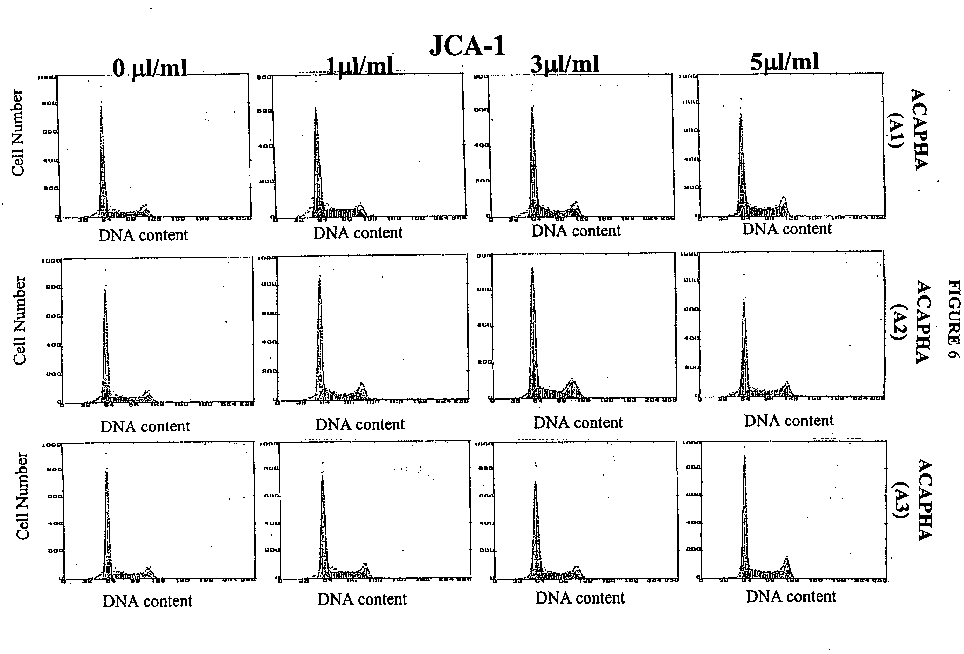

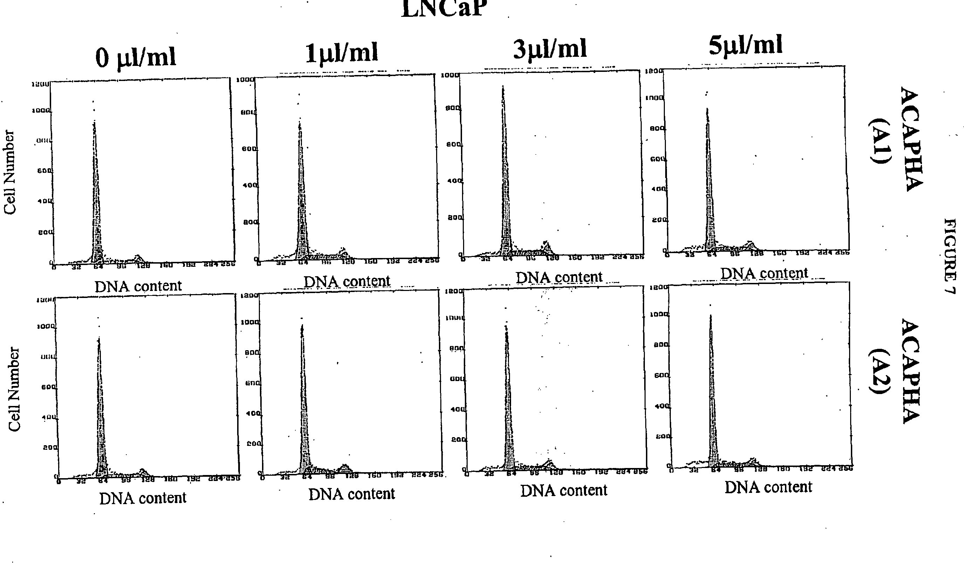

Effect of ACAPHA on Cell Cycle Progression. Cultures were exposed to varying concentrations of ACAPHA 1 (peripheral blood lymphocytes) or ACAPHA 1, 2 and 3 (JCA-1 and LNCaP cells). Cells were harvested, stained with DAPI fluorochrome and analyzed with a flow cytometer. ACAPHA 1 did not have a significant effect on cell cycle distribution of unstimulated and mitogen-stimulated peripheral blood lymphocytes. These results are presented in Tables 28 and 29. Figures 6 and 7 similarly show that ACAPHA 1 did not have a significant effect on cell cycle distribution of androgen-refractory human JCA-1 and androgen-responsive LNCaP cell lines. Further results are also shown in Tables 30 and 31. Using Jurkat (human T cell leukemia cell line) T cells and JCA-1 prostate cancer cells, ACAPHA 1 displayed differential growth suppressive effects on these two cell types, exerting minimal effects on T cell growth while substantially reducing growth of prostate cancer cells. This data suggests that ACAPHA has no apparent toxic effects. It was also demonstrated that the growth suppressive effect is unrelated to changes in p53 and p21 expression (Table 32).

**Table 28: Distribution of the various phases of the cell cycle**

| PBL | Time (hr) | ACAPHA 1 (µl/ml) | Cell Cycle Distribution | | |
|---|---|---|---|---|---|
| | | | G1 | S | G2/M |
| - PHA | 48 | 0 | 93.6 | 0.7 | 5.6 |
| - PHA | 48 | 1 | 95.3 | 0.5 | 4.2 |
| -PHA | 48 | 3 | 92.8 | 0.8 | 6.4 |
| - PHA | 48 | 5 | 96.6 | 0.7 | 2.7 |
| +PHA | 48 | 0 | 62.4 | 30 | 7.6 |
| +PHA | 48 | 1 | 63.1 | 29.6 | 7.3 |
| +PHA | 48 | 3 | 71.2 | 19.7 | 9.1 |
| +PHA | 48 | 5 | 73.8 | 19.5 | 6.7 |

**Table 29: Distribution of the various phases of the cell cycle**

| PBL | Time (hr) | ACAPHA 1 (µl/ml) | Cell Cycle Distribution | | |
|---|---|---|---|---|---|
| | | | G1 | S | G2/M |
| - PHA | 72 | 0 | 91.9 | 1.6 | 6.5 |
| - PHA | 72 | 1 | 91.7 | 1.4 | 6.9 |
| - PHA | 72 | 3 | 90.8 | 1.7 | 7.5 |
| - PHA | 72 | 5 | 89.7 | 1.8 | 8.5 |
| +PHA | 72 | 0 | 60.6 | 29.4 | 10 |
| +PHA | 72 | 1 | 53.8 | 35.3 | 10.8 |
| +PHA | 72 | 3 | 52.8 | 36.1 | 11.1 |
| +PHA | 72 | 5 | 56.8 | 31.8 | 11.4 |

**Table 30: Shows the quantitation of the effects of various concentrations of ACAPHA 1 (A1), ACAPHA 2 (A2) and ACAPHA 3 (A3) respectively on changes in the distribution of cell cycle phase progression in the androgen-refractory human prostate JCA-1 cell line. Cells were treated with ACAPHA for 72 hours.**

| JCA-1 | Treatment (µl/ml) | G1 | S | G2/M | Apoptosis |
|---|---|---|---|---|---|
| control | | 61.3 | 30.2 | 8.5 | |
| ACAPHA-A1 | 1 | 60.9 | 32.9 | 6.1 | |
| ACAPHA-A 1 | 3 | 54.8 | 38.2 | 7 | |
| ACAPHA-A 1 | 5 | 50.3 | 41.1 | 8.6 | |
| ACAPHA-A2 | 1 | 55.4 | 35.6 | 9 | |
| ACAPHA-A2 | 3 | 55.9 | 26 | 18.1 | |
| ACAPHA-A2 | 5 | 58 | 31.7 7 | 10.2 | |
| ACAPHA-A3 | 1 | 59.2 | 31.7 | 9.1 | |
| ACAPHA-A3 | 3 | 61.9 | 28.9 | 9.2 | |
| ACAPHA-A3 | 5 | 52.6 | 34.9 | 12.4 | |

**Table 31: The Quantitation of the effects of various concentrations of ACAPHA 1 (A1) and ACAPHA 2 (A2) respectively on changes in the distribution of cell cycle phase progression in the androgen-responsive human prostate LNCaP cells. Cells were treated with ACAPHA for 72 hours.**

| LNCaP | Treatment (µl/ml) | G1 | S | G2/M | Apoptosis |
|---|---|---|---|---|---|
| control | | 81.7 | 13.6 | 4.7 | |
| ACAPHA-A 1 | | 76.4 | 18 | 5.6 | |
| ACAPHA-A 1 | 3 | 75.3 | 16.9 | 7.8 | |
| ACAPHA-A 1 | 5 | 74.6 | 17.9 | 7.5 | |
| ACAPHA-A2 | 1 | 73.6 | 16.7 | 9.7 | |
| ACAPHA-A2 | 3 | 75 | 17.9 | 7.1 | |
| ACAPHA-A2 | 5 | 75.9 | 18 | 6.2 | |

**Table 32: Effect of ACAPHA on p53 and p12 Levels**

| | | |
|---|---|---|
| Treatment | p21 | p53 |
| Control | 17.8 | 16.2 |
| ACAPHA-1 (3µl/ml) | 18.1 | 16.6 |
| ACAPHA-2 (3µl/ml) | 18.1 | 16.1 |

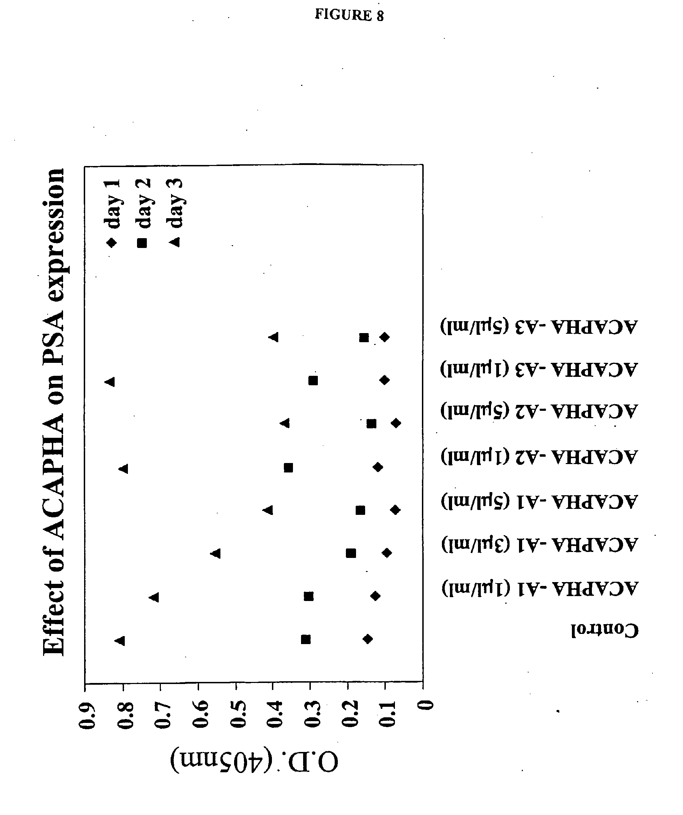

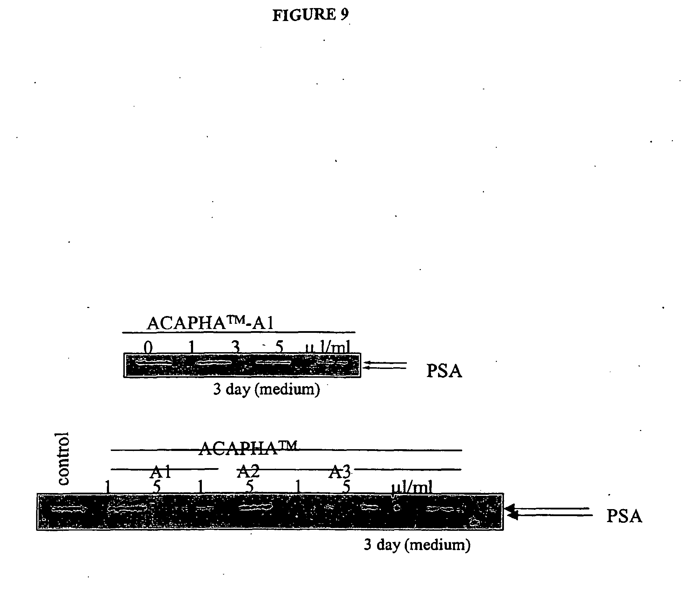

Effect of ACAPHA on Secreted Form of Prostate Specific Antigen (PSA). PSA has been identified as a marker to aid in the diagnosis of prostate cancer and for monitoring their responses to therapy. Figure 8 illustrates the effect of three formulations of ACAPHA, ACAPHA 1 (A1), ACAPHA 2 (A2) and ACAPHA 3 (A3), on time and dose-dependent PSA secretion in androgen-responsive LNCaP cells. The figure illustrates that increasing concentrations of ACAPHA significantly inhibited the secretion of PSA in both a dose and time-dependent manner. These results were confirmed by western blot analysis (Figure 9). Since PSA is a commonly accepted marker for human prostate cancer, these findings suggest that ACAPHA may be potentially useful in alleviating some of the symptoms of prostate carcinogenesis. Since the expression of PSA is downstream of changes in the androgen receptor, cellular androgen receptors and PSA in both control and ACAPHA treated cell extracts were evaluated with immunoblot analysis. Intracellular androgen receptors and PSA expression was down-regulated after treatment with 5 g/ml of ACAPHA extract (Figure not shown).

In summary, this cell line study indicates that ACAPHA 1 (A1), ACAPHA 2 (A2) and ACAPHA 3(A3) appear to inhibit the growth of both androgen sensitive and androgen refractory prostate cancer cells *in vitro.* The inhibitory effect does not appear to be related to changes in cell cycle progression, changes in p53 and p21 tumor suppressor gene expression or cellular apoptosis. Further ACAPHA 1 (A1), ACAPHA2 (A2) and ACAPHA 3(A3) inhibited the secretion of PSA tumor marker in *vitro.* ACAPHA is relatively benign and is not cytotoxic to normal cells. The mechanism of ACAPHA has not yet been elucidated, but it is likely related to genes that are directly or indirectly under the control of NFkB.

### EXAMPLE 8: In Vitro Inhibition of Proliferation of Human Leukemia Cells and Breast Cancer Cells by ACAPHA 2

Human Jurkat and K562 leukemia cells were maintained in RPMI1640 medium supplemented with 10% fetal bovine serum, glutamine and gentamycin. Human MCF-7 and MDA-MB-468 breast cancer cells were obtained from the American Type Culture Collection (Rockville, MD). MCF-7 cells were cultured as monolayers in Dulbecco=s MEM supplemented with 10% fetal bovine serum, glutamine and gentamycin. MDA-MB-468 cells were maintained in L15 medium supplemented with 10% fetal bovine serum, L-glutamine, sodium pyruvate, 2-mercaptoethanol and gentamycin. Jurkat, K562 and MCF-7 cells were cultured in 5% CO₂ humidified incubator at 37□C without CO₂.

ACAPHA 2 tablets were dissolved in 70% ethanol (340 mg/ml) and stirred with intermittent mixing at 150 rpm for 60 minutes at room temperature. The insoluble material was removed by centrifugation and the soluble supernatant was sterilized by passing through at 0.22 mm filter and kept in aliquots in a refrigerator. Before use, the stock was diluted further with culture medium to give the final indicated concentrations.

### Cell Growth

Quadruplicate or triplicate samples of cells in 96 well plate were treated with ACAPHA 2 prepared in culture medium at different concentrations. Cell growth was enumerated by counting viable cells in a hemacytometer by trypan blue dye exclusion on days 1, 2, 3 and 4. In the initial experiments it was established that Jurkat and K562 cells have logarithmic growth at days 2 and 3 while MCF-7 and MDA-MB-468 cells at day 4. In subsequent experiments Jurkat and K562 cells were assessed on day 2 while MCF-7 and MDA-MB-468 cells on day 4. Cell growth data from treated cells were normalized to percent of control.

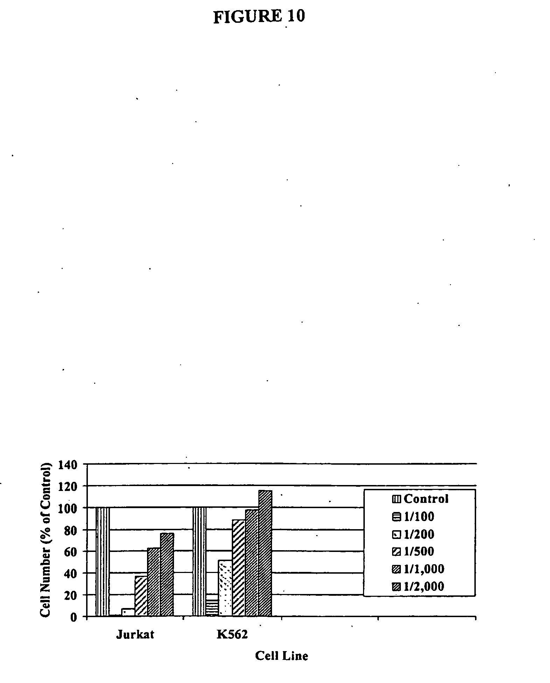

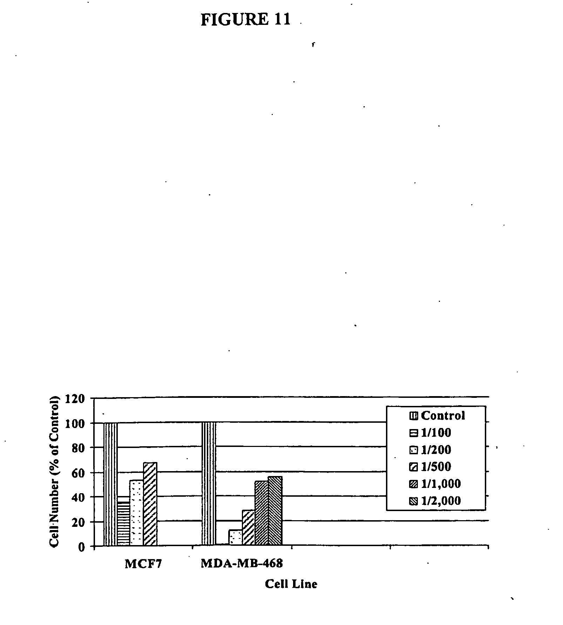

ACAPHA 2 at 1/100 dilution was growth inhibitory, causing an >50% of growth inhibition of both human leukemia (Jurkat and K5652; Figure 10) and breast cancer (MCF-7 and MDA-MB-468; Figure 11) cell lines.

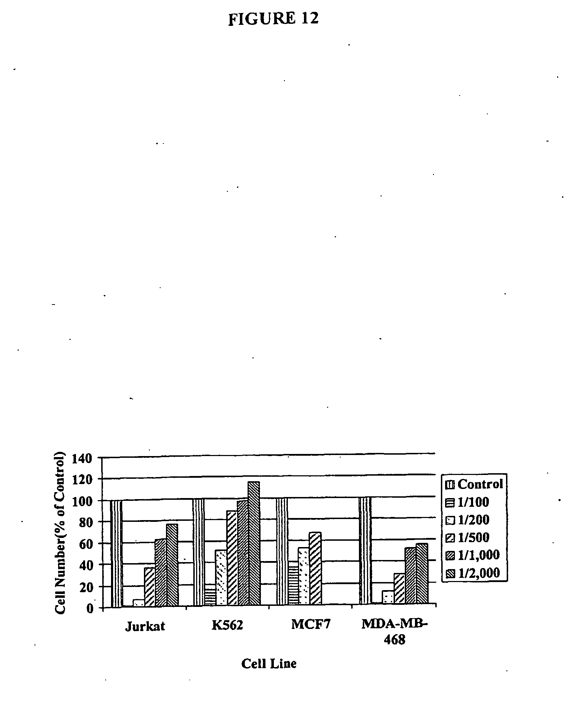

At higher dilutions, ACAPHA 2 showed differential growth inhibition on the cell lines. At 1/200 dilution, it caused 50% inhibition on K562 and MCF-7 cells but 93 and 87% inhibition on Jurkat and MDA-MB-468 cells respectively. At 1/500 dilution, ACAPHA 2 treatment still resulted in 63% and 72% growth inhibition on Jurkat and MDA-MB-468 cells respectively (Figures 12).

### Cell Cycle Analysis with Flow Cytometry

For Jurkat and K562 cells, at the end of the ACAPHA 2 treatment period, the cells were washed twice with Ca²⁺ and Mg²⁺ free Hanks= balanced salt solution, fixed in ice-cold 70% methanol overnight at -20□C. Fixed cells were washed free of methanol and treated with 2 mg/ml propidium iodide. Cell samples were passed through a 40 micron nylon mesh and a 27g needle before they were analyzed with a BD Bioscience Facscalibur flow cytometer. For adherent MCF-7 and MDA-MB-468 cells, they were detached from culture vessel with trypsin-EDTA solution before treated with the above procedure for cell cycle analysis.

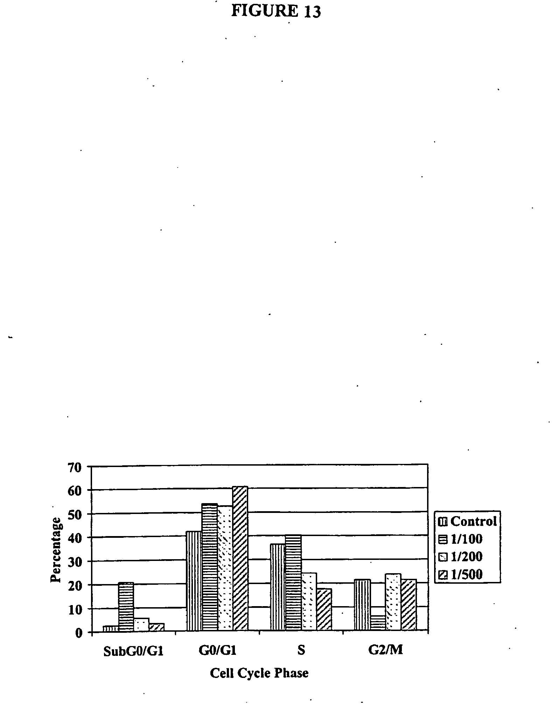

In Jurkat cells, ACAPHA 2 at 1/100 dilution caused an increase of cells in SubGO/G 1 phase (apoptotic/necrotic cells), cells arrested in GO/G 1 phase and reduced G2/M fraction. At 1/200 and 1/500 dilutions, ACAPHA 2 treatment still resulted in an increased number of cells arrested in G0/G1 phase and reduced of cells in S phase (Figure 13).

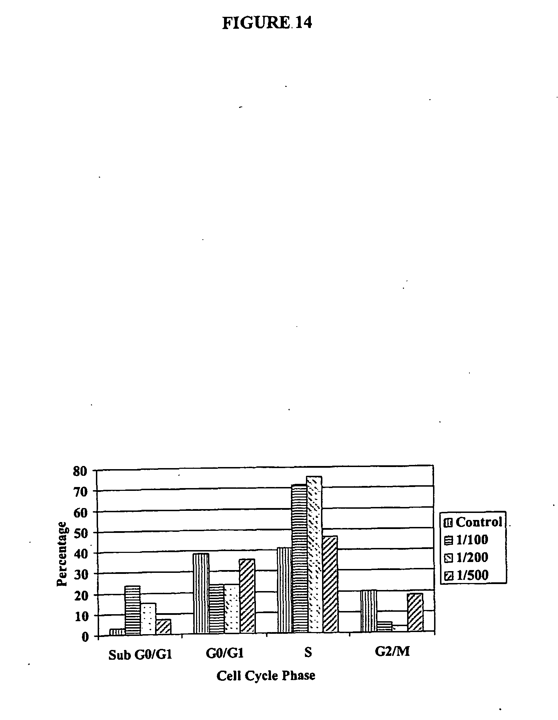

In K562 cells, ACAPHA 2 at 1/100 and 1/200 dilutions caused an increase in the number of cells in SubG0/G1 phase, reduction of cells in G0/G1 and G2/M phases, and increase of cells in S phase. The cell cycle profile of cells treated with 1/500 of ACAPHA 2 was similar to the control (Figure 14).

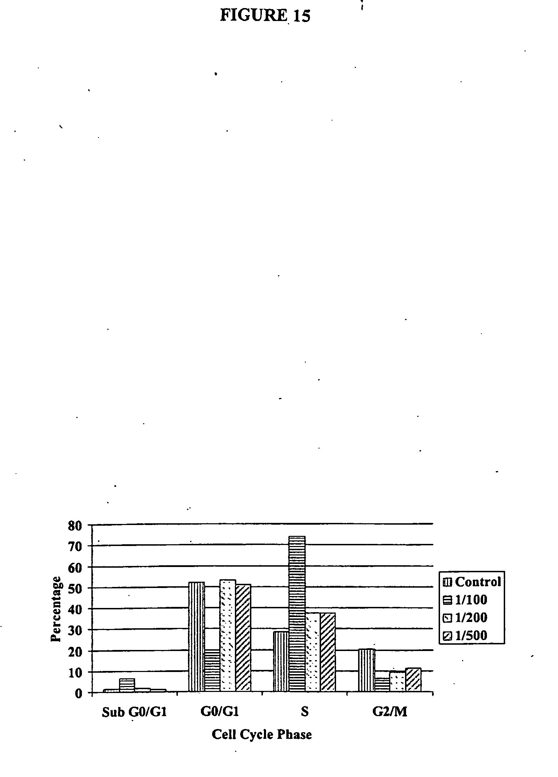

In MCF-7 cells, ACAPHA 2 at 1/100 dilution caused a slight increase of Sub0/G1 fraction, significant reduction of cells in G0/G1 and G2/M phases and significant increase of cells in S phase. At 1/200 and 1/500 dilutions, a slight increase of cells in S phase and decrease of cells in G2/M phase was observed (Figure 15).

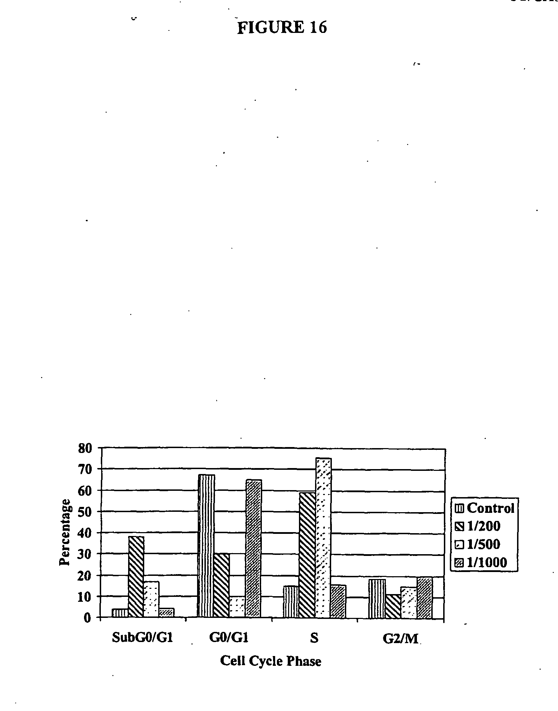

In MDA-MB-468 cells, ACAPHA at 1/100 was cytotoxic. At 1/200 dilution, ACAPHA caused a significant increase of cells in SubG0/G1 and S phases, and a significant reduction of cells in G0/G1 and G2/M phases. At 1/1,000 dilution, the distribution of cells in the different phases was similar to the control (Figure 16).

### C. DNA Fragmentation Analysis

DNA strand fragmentation was examined by extracting cellular DNA in the cell samples and subjecting them to 1% agarose gel electrophoresis in 40 mM Tris-acetate, 1 mM EDTA buffer pH 7.2 at 10 V/cm.

The results showed that ACAPHA did not cause DNA fragmentation in Jurkat and MCF-7 cells at 1/100 dilution by 1 % agarose gel electrophoresis suggesting ACAPHA did not induce apoptosis in these cells (Figures not shown).

### EXAMPLE 9: Use of ACAPHA 1 as an Adjunctive Therapy for Treatment of Esophageal Cancer

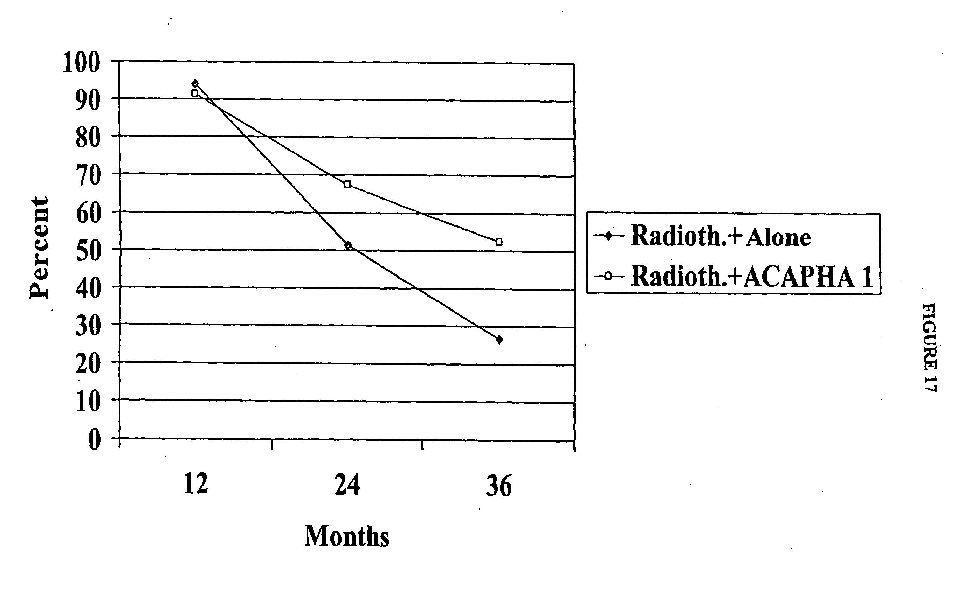

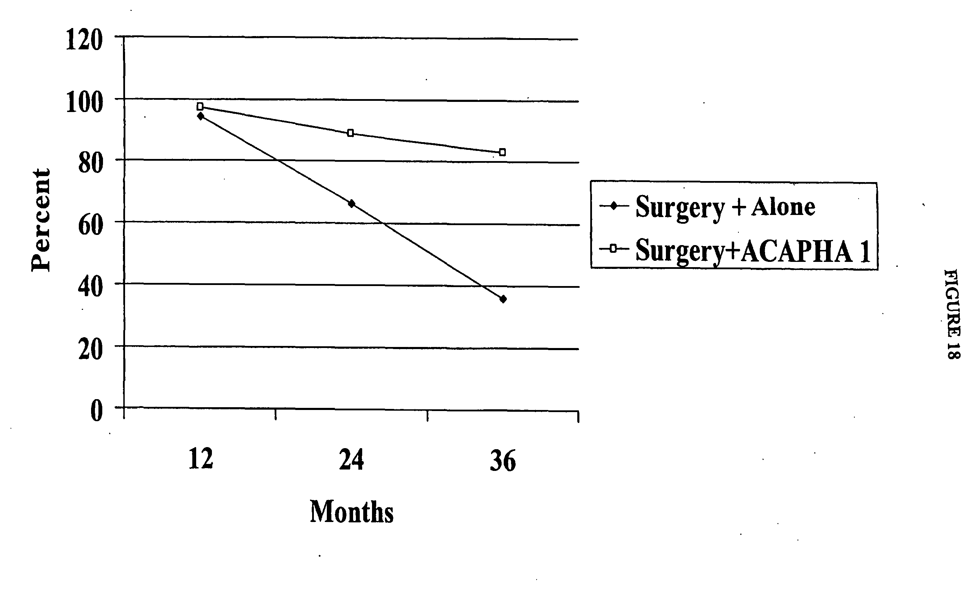

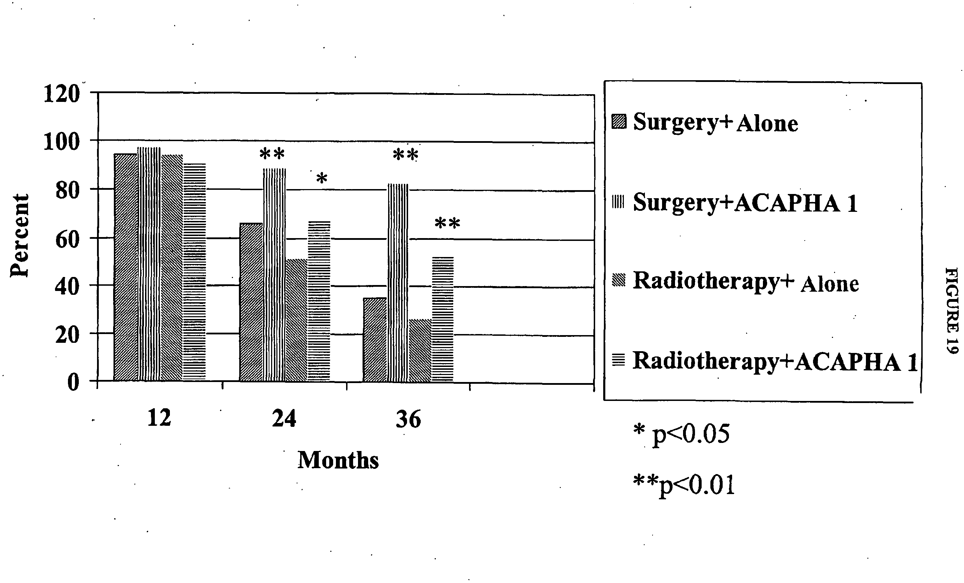

A five year trial in China commenced in January, 1997 to assess the efficacy of ACAPHA 1 as an adjunctive therapy for treatment of esophageal carcinoma. According to this trial, patients diagnosed with esophageal carcinoma were either treated by surgery or radiotherapy in conjunction with ACAPHA 1 administration, at a dose of 300mg tablets given 6 to 8 times per day. Historical data of patients treated for esophageal cancer by surgery or radiotherapy at the same cancer hospital was used for comparison. Survival rates in patient subjects twelve, twenty-four and thirty-six months after treatment commencement are summarized in Figures 17, 18 and 19 and in Tables 33 and 34. As shown in the Figures, the highest survival rate was in patients receiving ACAPHA 1 treatment in combination with surgery. Patients receiving ACAPHA 1 treatment in combination with radiotherapy also had a significantly higher survival rate as compared to patients receiving radiotherapy alone. The difference in survival rate between patients receiving conventional treatment and patients receiving ACAPHA 1 adjunctive therapy becomes more pronounced the longer the time period elapsed. For example, three years post-surgery, the survival rate of the 104 patients receiving surgery only was only 35.8%. By comparison, 82.9% of the 123 patients receiving a combination of surgery and ACAPHA 1 adjunctive therapy survived to the three year milestone.

**Table 33: Summary of clinical data comparing esophageal cancer staging in patients treated with ACAPHA 1 as an adjunct to conventional surgery or radiotherapy**

| Group | N | Stage I | | Stage | II | Stage | III | Stage | IV | Statistical Analysis |
|---|---|---|---|---|---|---|---|---|---|---|
| | | N | % | N | % | N | % | N | % | |
| Surgery + ACAPHA 1 | 123 | 7 | 5.7 | 47 | 38.2 | 63 | 51.2 | 6 | 4.9 | χ²=7.72 p>0.05 |
| Surgery Alone | 104 | 3 | 2.9 | 51 | 49.0 | 50 | 48.1 | C | C | |
| Radiotherapy + ACAPHA 1 | 9487 | 2 | 2.1 | 37 | 39.4 | 48 | 52.1 | 6 | 6.4 | χ²=1.41 p>0.05 |
| Radiotherapy Alone | | 7 | 8.1 | 27 | 31.0 | 53 | 60.9 | C | C | |

**Table 34: Comparison of survival rates of esophageal cancer patients treated with ACAPHA 1 as an adjunct to conventional surgery and radiotherapy 36 months post-treatment**

| Treatment | Months Post-Rx | Survival Rate (%) Plus ACAPHA 1 | | U-Test |
|---|---|---|---|---|
| Surgery | 122436 | 97.2 | 94.2 | U=1.09 p>0.05 |
| | | 88.9 | 66.1 | U=3.82 p<0.01 |
| | | 82.9 | 35.8 | U=6.04 p<0.01 |
| Radiotherapy | 122436 | 91.3 | 94.2 | U=0.74 p>0.05 |
| | | 67.2 | 51.3 | U=2.56 p<0.05 |
| | | 52.3 | 26.4 | U=2.71 p<0.01 |

In a further clinical trial started in January 1995, the survival rate after five years of post-radiotherapy patients with esophageal cancer also treated with ACAPHA was determined. Patients were randomized into two equal groups; 140 patients after radiotherapy for esophageal cancer were randomized to the treatment group and 140 patients after radiotherapy esophageal cancer at the same time were randomized to the control group. For patients in the ACAPHA treatment group, 6 to 8 tablets of ACAPHA were given twice a day. The effect of ACAPHA on the survival rate of the post-surgery patient with esophageal cancer is shown in Table 35. The one, two, three and four year survival rate in the treatment group were 76.12%, 45.52%, 39.55% and 29.1% respectively, which were 13.3%, 16.9%, 32.9% and 52.5% higher than the 67.18, 38.93%, 29.77% and 19.08% in the control group. Though there was no significant difference between each group (P>0.05, Table 35), the survival rate was increasing yearly. After 5 years, the survival rate in treatment group was 26.87%, which was 85.3% higher than the 14.5% in the control group (p<0.01). It indicates that ACAPHA can increase the 5 year survival rate of post radiotherapy patients with esophageal cancer and has good results as an adjuvant to radiotherapy for patients with esophageal cancer.

**Table 35. Effect of ACAPHA on post radiotherapy survival rate of esophageal cancer patients.**

| **Survival Year** | **ACAPHA** | | **Control** | | **U Value** | | **Increase in Survival Rate (%)** |
|---|---|---|---|---|---|---|---|
| | **Survival Rate** | **SD** | **Survival Rate** | **SD** | | | |
| 1 | 0.7612 | 0.0368 | 0.6718 | 0.0410 | 1.6227 | p>0.05 | 13.3 |
| 2 | 0.4552 | 0.0430 | 0.3893 | 0.0426 | 1.0887 | p>0.05 | 16.9 |
| 3 | 0.3955 | 0.0422 | 0.2977 | 0.0400 | 1.6820 | p>0.05 | 32.9 |
| 4 | 0.2910 | 0.0392 | 0.1908 | 0.0343 | 1.9237 | p>0.05 | 52.5 |
| 5 | 0.2687 | 0.0383 | 0.1450 | 0.0308 | 2.5169 | p<0.01 | 85.3 |

The data of the two studies of chemoprevention with ACAPHA after radiotherapy were combined. The results indicated that the distribution of the samples were comparable. Table 36 shows that one, two, three, four and five year survival rate in the treatment group were 78.93%, 51.68%, 44.93%, 33.23% and 30.67%, which were 14.7%, 35.7%, 59.7%, 74.1% and 111.3% higher than the 68.81%, 38.07%, 28.14%, 19.09% and 14.51% in the control group (p<0.01). The survival rates increased yearly.

**Table 36. Effect of ACAPHA on post radiotherapy survival rate of esophageal cancer patients.**

| **Survival Year** | **ACAPHA** | | **Control** | | **U Value** | | **Increase in Survival Rate (%)** |
|---|---|---|---|---|---|---|---|
| | **Survival Rate** | **SD** | **Survival Rate** | **SD** | | | |
| 1 | 0.7893 | 0.0279 | 0.6881 | 0.0314 | 2.4093 | p<0.01 | 14.7 |
| 2 | 0.5168 | 0.0354 | 0.3807 | 0.0329 | 2.8162 | p<0.01 | 35.7 |
| 3 | 0.4493 | 0.0367 | 0.2814 | 0.0306 | 3.2138 | p<0.01 | 59.7 |
| 4 | 0.3323 | 0.0383 | 0.1909 | 0.0287 | 2.9545 | p<0.01 | 74.1 |
| 5 | 0.3067 | 0.0381 | 0.1451 | 0.0272 | 3.4401 | p<0.01 | 111.3 |

Based on the above results, it can be concluded that ACAPHA could increase the survival rate of the post treatment (radiotherapy or surgery) patients with esophageal cancer.

### EXAMPLE 10: Effect of ACAPHA 1 on Helicobacter pylori Infection Associated Polyp Type Gastritis

Polyp type gastritis is a special form of chronic gastritis. It can be present as the only problem, or it can exist with other gastric disorders. The special feature is the presence of pathological umbilicus shaped swellings of the gastric mucosa. In China, it comprises about 15% of chronic gastritis and 85% of these patients have *H. pylori* (HP) infection.

*H. pylori* is important in the etiology of gastric cancer. It has recently been recognized by the International Agency for Research on Cancer as a human carcinogen. HP is assumed to cause gastric cancer indirectly because it provokes gastritis, which is a precursor of gastric atrophy, metaplasia and dysplasia. In the following example, the effect of ACAPHA 1 on HP infection associated polyp type gastritis was investigated.
A total of 16 patients, ages ranging from 35 to 62 years old, were used in this study. Disease duration ranged from 4 to 12 months, with a mean of 7 months. Selection criteria of polyp type gastritis was based on diagnostic criteria and HP infection was tested using urease test and biopsy sections.

An experienced endoscopist examined each patient prior to selection. Detailed descriptions were made during the endoscopic assessment and photos were taken. In addition, a minimum of 2 mucosal specimens in areas with pathological changes were collected and examined by an experienced pathologist. Patients that fulfilled the criteria for polyp type gastritis and tested positive for HP infection were included in the study group. The detailed clinical condition of the test subjects were then recorded and WBC counts and liver and kidney function tests were performed.
Patients in the study group were instructed to take 8 tablets of ACAPHA 1, twice a day for 3 months (a total of 16 tablets /day). During the treatment, all other medications used for treatment of gastric conditions were stopped. At the end of the treatment period, side effects of ACAPHA 1 and changes in the disease states were recorded. WBC, liver and kidney function tests were repeated. The same endoscopist repeated the gastric endoscopic examinations, recording any observable changes and taking photos. Biopsy samples were taken in the same mucosal areas as before and the same pathologists assessed the histological sections. The presence of HP infection was again tested.

None of the 16 patients had adverse reactions or side effects during treatment with ACAPHA 1. Among the treated patients, 13 cases (81.3%) had obvious improvement in the original clinical symptoms of gastric distention, intestinal pain, heart burn due to hypersecretion of gastric acid and vomiting after 1 month of ACAPHA 1 treatment. After 2 months of treatment, the original clinical symptoms disappeared in these patients. After 3 months of treatment, only 2 cases showed no significant improvement in clinical symptoms. The efficacy rate as assessed by clinical symptoms was 87.5%.

After 3 months of ACAPHA 1 treatment, endoscopic examination revealed that in 12 of the 16 cases, the pathological features of mucosal umbilicus type swelling had totally disappeared. Histopathological assessment showed that there was regression from polyp type gastritis to a mild form of superficial gastritis. In addition, 11 cases tested negative for HP infection. There were 4 cases that did not respond to treatment but after an additional 3 months of ACAPHA 1 treatment, 3 patients no longer showed gastric mucosal umbilicus type swelling. Histopathological assessment of these patients showed a regression to a mild form of superficial gastritis. In 2 of the 4 cases, HP infection tested negative. The overall efficacy rate of ACAPHA 1 treatment as assessed by gastric endoscopy and histopathology was 93.8%. The HP infection curative rate was 81.3%.

The results showed that ACAPHA 1 was an effective treatment for polyp type gastritis as measured by clinical assessment, endoscopic examination and histopathological assessment. In addition, ACAPHA I was effective in eradicating HP infection. The eradication rate of HP infection by ACAPHA 1 is similar to the rate achieved by the internationally recognized standard treatment of HP using a double drug regimen, consisting of bismuth compound and an antibiotic, or bismuth-based triple therapy.

### EXAMPLE 11: Effect of ACAPHA 1 on Chronic Atrophic Gastritis

Chronic atrophic gastritis is a common disease in middle and old age. The etiology and mechanism of the disease is still unclear. As a result, there is still no specific effective therapy and current treatment is mainly directed at treatment of symptoms.

In chronic atrophic gastritis, gastric mucosal epithelial gland metaplasia and atypical hyperplasia are characteristic pathological features, which are also closely associated with the development of gastric carcinoma. Some scientists have even considered that these features are a precancerous pathological change.

Intestinal gland metaplasia refers to the presence of intestinal glandular epithelium in the gastric mucosa. Based on the amount present, the disease can be classified into mild, moderate and severe categories.

Atypical hyperplasia of gastric mucosal epithelium is also referred to as gastric dysplasia. The main features include atypical morphology of the cells, irregular architecture and atypical cellular distribution. According to the degree of tissue involvement the changes can also be classified into mild, moderate and severe categories. Due to the absence of specific effective drugs to improve or cure this condition, surgery is usually recommended to prevent the development of cancer in severe cases of dysplasia. Development of a new drug that can improve or eradicate intestinal gland metaplasia and dysplasia should greatly reduce the incidence of gastric carcinoma. In this example, the effect of ACAPHA I on chronic atrophic gastritis was investigated.

A total of 26 subjects were used in this study conducted in China. All patients were diagnosed with chronic atrophic gastritis and had either intestinal gland epithelium metaplasia or dysplasia. Selection of patients was carried out according to strict international diagnostic criteria. The severity of gastric mucous gland epithelium metaplasia and dysplasia was also assessed according to strict international criteria.

Before selection, an experienced endoscopist examined the gastric lining of each patient and obtained gastric mucosal biopsy samples at defined sites. An experienced pathologist then examined histological sections of the biopsy samples. Cases that fulfilled the selection criteria were selected for the study. Detailed case histories were then recorded for each patient and WBC, kidney and liver function tests were performed.

Patients who were admitted into the study were told to take 3 ACAPHA 1 tablets, twice a day for 3 months. All other medications for the treatment of chronic atrophic gastritis were stopped. At the end of the treatment period, adverse reactions and side effects caused by ACAPHA 1 and clinical changes were recorded. WBC, liver and kidney function tests were once again performed. The same endoscopist examined the gastric lining of the patients and recorded any observable changes. A repeat sampling of gastric mucosal biopsies were performed in the same sites as before and the same pathologist examined histological sections from the biopsies. Finally, an independent experienced pathologist reexamined each histological section to confirm the accuracy of the findings.

None of the study patients with chronic atrophic gastritis experienced adverse reactions or side effects after taking ACAPHA 1. In addition, there were no significant changes in the WBC, liver and kidney function tests after ACAPHA 1 therapy. In 16 cases (61.5%), clinical symptoms of abdominal pain, belching (eructation), and abdominal distention significantly improved after 1 month of ACAPHA 1 therapy. After 2 months, 22 cases (85.4%) showed significant improvement of clinical symptoms and after 3 months, only one patient did not have significant improvement. Therefore, the clinical symptom improvement rate was 96.2% after three months.

Comparison of endoscopic examinations before and after ACAPHA I treatment indicated that 7 cases with focal necrosis had significant improvement or disappearance of lesion following treatment. There were no significant changes in other features of chronic atrophic gastritis revealed by endoscopy.

Histopathological examination of biopsy sections before and after ACAPHA 1 treatment revealed significantly more than the endoscopic examinations. It was found treatment revealed significantly more than the endoscopic examinations. It was found that 7 cases of chronic atrophic gastritis regressed to chronic superficial gastritis. In particular, out of 12 cases of severe intestinal gland epithelium metaplasia, 5 regressed to moderate or mild forms of the metaplasia and 2 no longer showed any features of severe metaplasia. Out of 9 cases with moderate intestinal glandular epithelium metaplasia, 3 regressed to mild metaplasia and the remaining 6 no longer showed any features of moderate metaplasia. Finally, in all 5 cases of mild intestinal glandular epithelium metaplasia, all abnormal features disappeared after ACAPHA 1 treatment. In the cases of severe atypical hyperplasia, 4 cases regressed to mild atypical hyperplasia and in 2 cases the pathological changes disappeared. In all 8 cases with moderate atypical hyperplasia and 12 cases with mild atypical hyperplasia the abnormal features disappeared after ACAPHA 1 treatment.

The results from this study indicate that ACAPHA I was effective against chronic atrophic gastritis and intestinal gland epithelium metaplasia and dysplasia.

### EXAMPLE 12: The Effect of ACAPHA 1 on Oral Lichen Planus

Oral Lichen Planus (OLP) is a chronic inflammatory condition associated with the immune system. Some reports have suggested that it has a tendency to become cancerous. Presently there is no effective treatment for this condition. In this example, the effects of ACAPHA 1 on patients with OLP were investigated.

Ten patients with confirmed diagnoses of OLP and unresponsive to other forms of treatment were used in this study. One month prior to ACAPHA 1 treatment, all patients stopped taking medication that may affect the immune system. ACAPHA 1 was given at a dose of 3 tablets, 3 times a day for 4 to 12 months. The patients were given oral examinations every 2 months and had serum and urine tests for SGPT and BUN. Following ACAPHA 1 treatment, OLP in the patients was monitored for another 4 to 7 months.

The overall effective rate was 90%, with 10% cured, 40% showing marked improvement, 40% showing improvement and 10% showing no change. The liver and kidney functions remained normal during the study indicating that ACAPHA 1 did not have any detectable side effects.

The results suggest that ACAPHA 1 is an effective treatment for OLP. In view of the fact that the treatment is simple and inexpensive and long term treatment did not affect liver and kidney functions, this would suggest that ACAPHA 1 has potential as a treatment for OLP and to prevent it from progressing to oral cancerous lesions.

### EXAMPLE 13: Effect of ACAPHA 2 on Breast Cystic Hyperplasia

The breast cystic hyperlasia is common in clinical practice, especially in married women or those with child-bearing history. Based on ACAPHA 2's antiproliferative function on the epithelium, improving the immune response and regulating endocrine function, 121 patients were chosen for this study. The results of ACAPHA 2 on breast cystic hyperplasia are shown below in Table 37.

**Table 37. Effect of ACAPHA 2 on mammary cystic hyperplasia**

| Symptom and Sign | No. of Cases | Cured(%) | Improved(%) | Ineffective(%) |
|---|---|---|---|---|
| Pain | 121 | 72(59.5) | 36(29.6) | 12(9.9) |
| Node | 121 | 68(56.2) | 49(40.5) | 4(3.3) |

Pathologically, proliferation can be seen in the fibrous tissue around the lobe of the mammary gland and some papillary proliferation can be seen in the epithelium. Symptoms are pains in the breast periodically and persistently, nodular mass with different size and unclear edge. After treatment with ACAPHA 2, the efficacy is satisfactory without any obvious side effects.

### EXAMPLE 14: Long Term Effect of ACAPHA (8 Years) on Mortality & Morbidity of Cancers (1992-2001)

Patients ages 40 to 65 diagnosed with severe epithelial dysplasia of esophagus by cytology, were randomized into 2 groups. The treatment group was given 8 tablets daily of ACAPHA I and the control group was given a placebo. The study was conducted for three years with a follow up after treatment for an additional five years. ACAPHA 1 was shown to have a significant positive effect on the incidence and mortality of esophageal cancer and other cancers.

Table 38 shows the incidence of cancer in patients receiving ACAPHA 1 for three years after a five year follow up.

**Table 38**

| | **Control group** | **Treatment group** | **Sub-total** |
|---|---|---|---|
| **Esophagus** | 207 | 55 | 262 |
| **Other cancers** | | | |
| Lung | 21 | 4 | 25 |
| Liver | 23 | 5 | 28 |
| Stomach | 10 | 3 | 13 |
| Intestine | 2 | 3 | 5 |
| Larynx | 2 | 2 | 4 |
| Oral | 1 | 0 | 1 |
| Pancreas | 1 | 0 | 1 |
| Penis | 2 | 0 | 2 |
| Cervix | 3 | 1 | 4 |
| Ovary | 1 | 0 | 1 |
| Breast | 2 | 0 | 2 |
| Bone | 0 | 1 | 1 |
| Brain | 4 | 0 | 4 |
| Thyroid | 1 | 0 | 1 |
| Lymphoma | 2 | 0 | 2 |
| Unknown | 0 | 1 | 1 |
| Sub-Total | 75 | 20 | 95 |

Table 39 shows the effect of ACAPHA 1 on mortality and morbidity.

**Table 39**

| Group | # of cases | Deceased case | | | Diagnosed case | | | Sub-total | | |
|---|---|---|---|---|---|---|---|---|---|---|
| | | # of cases | % | PR(95%CI) | # of cases | % | RR(95%CI) | # of Cases | % | RR(95%CI) |
| Control | 2826 | 321 | 11.4 | RR=0.70 | 115 | 4.07 | RR=0.43 | 436 | 15.43 | RR=0.60 |
| | | | | (0.55-0.87) | | | (0.27-0.67) | | | (0.49-0.75) |
| Treatment | 1242 | 101 | 8.13 | | 22 | 1.77 | | 123 | 9.9 | |

Table 40 shows the effect of ACAPHA 1 on mortality and incidence of esophageal cancer.

**Table 40**

| Group | # of cases | Deceased case | | | Diagnosed case | | | Sub-total | | |
|---|---|---|---|---|---|---|---|---|---|---|
| | | # of cases | % | RR(95%CI) | # of cases | % | RR(95%CI) | # of Cases | % | RR(95%CI) |
| Control | 2826 | 66 | 2.34 | RR=0.65 | 9 | 0.32 | RR=0.25 | 75 | 2.64 | RR=0.60 |
| | | | | (0.39-1.09) | | | (0.03-0.40) | | | (0.37-0.99) |
| Treatment | 1242 | 19 | 1.53 | | 1 | 0.1 | | 20 | 1.61 | |

Table 41 shows the effect of ACAPHA 1 on mortality of cancers other than esophageal cancer.

**Table 41**

| Group | # of cases | Deceased case | | | Diagnosed case | | | Sub-total | | |
|---|---|---|---|---|---|---|---|---|---|---|
| | | # of cases | % | RR(95%CI) | # of cases | % | RR(95%Cl) | # of Cases | % | RR(95%Cl) |
| Control | 2826 | 66 | 2.34 | RR=0.65 | 9 | 0.32 | RR=0.25 | 75 | 2.64 | RR=0.60 |
| | | | | (0.39-1.09) | | | (0.03-0.40) | | | (0.37-0.99) |
| Treatment | 1242 | 19 | 1.53 | | 1 | 0.8 | | 20 | 1.61 | |

Table 42 shows the effect of ACAPHA 1 on incidence of mortality of cardiovascular diseases.

**Table 42**

| Group | # of cases | Deceased case | | | Diagnosed case | | | Sub-total | | |
|---|---|---|---|---|---|---|---|---|---|---|
| | | # of cases | % | RR(95%CI) | # of cases | % | RR(95%CI) | # of Cases | % | RR(95%CI) |
| Control | 2826 | 33 | 1.17 | RR=0.69 | 21 | 0.74 | RR=0.22 | 54 | 1.91 | RR=0.50 |
| | | | | (0.39-1.40) | | | (0.05-0.92) | | | (0.27-0.94) |
| Treatment | 1242 | 10 | 0.81 | | 2 | 0.16 | | 12 | 0.97 | |

Table 43 shows the effect of ACAPHA 1 on the incidence and mortality of cerebral vascular diseases.

**Table 43**

| Group | # of cases | Deceased case | | | Diagnosed case | | | Sub-total | | |
|---|---|---|---|---|---|---|---|---|---|---|
| | | # of cases | % | RR(95%Cl) | # of cases | % | RR(95%Cl) | # of Cases | % | RR(95%Cl) |
| Control | 2826 | 48 | 1.7 | RR=0.71 | 12 | 0.43 | RR=0.76 | 60 | 2.12 | RR=0.72 |
| | | | | (0.40-1.27) | | | (0.24-2.35) | | | (0.43-1.21) |
| Treatment | 1242 | 15 | 1.21 | | 4 | 0.32 | | 19 | 1.53 | |

| | | | | | | | | | | |
|---|---|---|---|---|---|---|---|---|---|---|
| Note (for tables 38 to 43) 1. ARR= 1@: no protective & improvement effect 2. ARR< 1 @: with protective effect 3. ARR> 1 @: with improvement effect 4. A95%Cl > 1.0@: without notable statistical difference | | | | | | | | | | |

### EXAMPLE 15: Long Term Effect of ACAPHA (18 Years) on Mortality & Morbidity of Cancers (1983-2001)

Patients aged 40 to 65 were diagnosed with severe epithelial dysplasia of esophagus by cytology, were randomized into two groups. The treatment group received ACAPHA 1 at a dose of 8 tablets per day. The control group received a placebo. The treatment period was for five years with a follow up for an additional 13 years. ACAPHA 1 was shown to have significant positive effect on the incidence and mortality of esophageal cancer and other cancers.

Table 44 shows the incidence of cancer in patients receiving ACAPHA I for five years and off for 13 years.

**Table 44**

| | **Control group** | **Treatment group** | **Sub-total** |
|---|---|---|---|
| **Esophagus** | 121 | 77 | 198 |
| **Other cancers** | | | |
| Lung | 3 | 3 | 6 |
| Liver | 6 | 3 | 9 |
| Stomach | 17 | 10 | 27 |
| Intestine | 3 | 1 | 4 |
| Larynx | 1 | 0 | 1 |
| Breast | 2 | 1 | 3 |
| Bone | 0 | 1 | 1 |
| Brain | 0 | 1 | 1 |
| Sub-Total | 32 | 20 | 52 |

Table 45 shows the effect of ACAPHA I on mortality and morbidity.

**Table 45**

| Group | # of cases | Deceased case | | | Diagnosed case | | | Sub-total | | |
|---|---|---|---|---|---|---|---|---|---|---|
| | | # of cases | % | RR(95%CI) | # of cases | % | RR(95%CI) | # of Cases | % | RR(95%CI) |
| Control | 523 | 199 | 38.05 | RR=0.60 | 74 | 14.15 | RR=0.38 | 273 | 52.20 | RR=0.45 |
| | | | | (0.46-0.78) | | | (0.24-0.59) | | | (0.35-0.57) |
| Treatment | 528 | 142 | 26.89 | | 31 | 5.87 | | 173 | 32.77 | |

Table 46 shows the effect of ACAPHA I on mortality and incidence of esophageal cancer.

**Table 46**

| Group | # of cases | Deceased case | | | Diagnosed case | | | Sub-total | | |
|---|---|---|---|---|---|---|---|---|---|---|
| | | # of cases | % | RR(95%Cl) | # of cases | % | RR(95%Cl) | # of Cases | % | RR(95%CI) |
| Control | 523 | 87 | 16.63 | RR=0.70 | 34 | 6.50 | RR=0.33 | 121 | 23.14 | RR=0.57 |
| | | | | (0.50-1.00) | | | (0.17-0.65) | | | (0.41-0.78) |
| Treatment | 528 | 65 | 12.31 | | 12 | 2.27 | | 77 | 14.58 | |

Table 47 shows the effect of ACAPHA 1 on mortality of cancers other than esophageal cancer

**Table 47**

| Group | # of cases | Deceased case | | | Diagnosed case | | | Sub-total | | |
|---|---|---|---|---|---|---|---|---|---|---|
| | | # of cases | % | RR(95%Cl) | # of cases | % | RR(95%Cl) | # of Cases | % | RR(95%Cl) |
| Control | 523 | 26 | 4.97 | RR=0.68 | 6 | 1.15 | RR=0.33 | 32 | 6.12 | RR=0.60 |
| | | | | (0.37-1.25) | | | (0.07-1.63) | | | (0.34-1.07) |
| Treatment | 528 | 18 | 3.41 | | 2 | 0.38 | | 20 | 3.79 | |

Table 48 shows the effect of ACAPHA 1 on incidence of mortality of cardiovascular diseases.

**Table 48**

| Group | # of cases | Deceased case | | | Diagnosed case | | | Sub-total | | |
|---|---|---|---|---|---|---|---|---|---|---|
| | | # of cases | % | RR(95%Cl) | # of cases | % | RR(95%Cl) | # of Cases | % | RR(95%Cl) |
| Control | 523 | 29 | 5.55 | RR=0.71 | 15 | 2.87 | RR=0.65 | 44 | 8.41 | RR=0.68 |
| | | | | (0.40-1.25) | | | (0.29-1.06) | | | (0.42-1.09) |
| Treatment | 528 | 21 | 3.98 | | 10 | 1.89 | | 31 | 5.87 | |

Table 49 shows the effect of ACAPHA I on incidence of mortality of cerebral vascular diseases.

**Table 49**

| Group | # of cases | Deceased case | | | Diagnosed case | | | Sub-total | | |
|---|---|---|---|---|---|---|---|---|---|---|
| | | # of cases | % | RR(95%CI) | # of cases | % | RR(95%CI) | # of Cases | % | RR(95%CI) |
| Control | 523 | 39 | 7.46 | RR=0.62 | 13 | 2.49 | RR=0.38 | 52 | 9.94 | RR=0.55 |
| | | | | (0.37-1.04) | | | (0.13-1.06) | | | (0.34-0.87) |
| Treatment | 528 | 25 | 4.74 | | 5 | 0.95 | | 30 | 5.68 | |

| | | | | | | | | | | |
|---|---|---|---|---|---|---|---|---|---|---|
| **Note (for tables 44 to 49)** 1. ARR=1@: no protective & improvement effect 2. ARR< 1@: with protective effect 3. ARR> 1@: with improvement effect 4. A95%Cl > 1.0@: without notable statistical difference | | | | | | | | | | |

### EXAMPLE 16: Effect of ACAPHA 1 on Bronchial Dysplasia

Bronchial dysplasia is a premalignant lesion of the bronchial epithelium. Currently there is no standard treatment for bronchial dysplasia. The prevalence of bronchial intraepithelial neoplasia (dysplasia and carcinoma in-situ) has been studied using sputum cytology examination and flourescence bronchoscopy. In smokers with chronic obstructive pulmonary disease (COPD) and a smoking history of more than 40 packs per year, the prevelence of mild, moderate, or severe atypia and canconoma in-situ in sputum cytology examinations was found to be 48%, 25%, 0.8% and 0.9% respectively. In current and former smokers over 40 years of age with a smoking history of more than 30 packs per year, the prevelance of mild, moderate or severe dysplasia and carcinoma in-situ on flourescence bronchoscopy and biopsy, was found to be 44%, 14%, 4.3% and 1.2% respectively. Because tobacco use is one of the major causes of lung cancer, and former heavy smokers retain an elevated risk for lung cancer even years after they have stopped smoking, the effects of ACAPHA 1 on bronchial dysplasia in former smokers was investigated.

Twenty current and former smokers over 40 years of age with a smoking history of at least 30 pack-years (i.e. 1 pack per day for 30 years or more) and one or more sites of bronchial dysplasia on fluorescence bronchoscopy directed bronchial biopsies were recruited to take part in the study. Participants from the placebo arm of two concurrent chemoprevention trials with identical inclusion and exclusion criteria and similar clinical protocols aside from the chemopreventive agent were used for comparison purposes.

Following an initial interview, which included a questionnaire to document the smoking history, fluorescence bronchoscopy using the LIFE-Lung device (Xillix Technologies Corp., Richmond, B.C., Canada) was performed on each patient. Biopsies were taken from areas with abnormal fluorescence. In addition, at least two control biopsies were taken from an upper or lower lobe. A minimum of four biopsies were taken per individual.

The participants were instructed to take ACAPHA 1 at a dose of 8 tablets, twice a day for 6 months. They were seen monthly for examination of drug related adverse effects. Complete blood counts, AST, LD, bilirubin, alkaline phosphatase, calcium, phosphate, electrolytes, BUN, creatinine, triglycerides and cholesterol were measured at baseline and monthly for 6 months while on ACAPHA 1 for toxicity monitoring. Fluorescence bronchoscopy was repeated after 6 months of ACAPHA 1 treatment. All previously biopsied sites were identified and re-biopsied under fluorescence bronchoscopy. Biopsies were also taken from new areas that appeared to be dysplastic under fluorescence examination.

The biopsies were fixed in buffered formalin, embedded in paraffin, sectioned and stained with hematoxylin and eosin. All the biopsies were systematically reviewed by an experienced pathologist. All biopsies were classified into one of eight groups. The normal group was represented by pseudostratified ciliated columnar epithelium. The basal cell hyperplasia group was represented by an increase in the number and stratification of normal-appearing basal cells still covered with normal ciliated or mucin secreting cells. The metaplasia group was represented by a stratified epithelium and cytoplasmic changes consistent with squamoid differentiation. Mild, moderate or severe dysplasia and carcinoma in-situ were classified according to WHO criteria. The final group was classified as invasive, metastatic carcinoma.

The primary end-point of the study (i.e. surrogate end-point biomarker) was change in the histopathology grade based on the risk of progression to invasive cancer. From the study by Frost and co-workers (J. Occp. Med 1986; 28:692-703), the risk of progression of mild, moderate and severe dysplasia to invasive lung cancer within nine years were 4%, 10%, and 40%, respectively. On a site by site analysis, complete response (CR) was defined by regression of the dysplastic lesion to hyperplasia/normal. Progressive disease (PD) was defined as appearance of lesions that were mild dysplasia or worse irrespective of whether the site was biopsied at baseline or worsening of the dysplastic lesions present at baseline by two grades or more (i.e. mild dysplasia to severe dysplasia or worse, moderate/severe dysplasia to carcinoma in-situ/invasive cancer). No response (NR) referred to sites that were not a CR or PD.

On a participant by participant basis, CR was defined as regression of all dysplastic lesions found at baseline to no higher than hyperplasia as defined by the site by site analysis at six months and no appearance of new dysplastic lesions that were mild dysplasia or worse. PD was defined as progression of one or more sites to a higher grade as defined by the site by site analysis referred to above or appearance of new dysplastic lesions that were mild dysplasia or worse at six months. Partial response (PR) was defined as regression of some but not all of the dysplastic lesions but no appearance of new lesions that were mild dysplasia or worse. No response (NR) referred to subjects who did not have a CR, PR or PD.

Descriptive statistics were used to summarize subject characteristics and pathologic evaluations of the bronchial biopsy examinations. Comparison between groups was done with the Mann-Whitney test. Pearson's Chi-square test was applied to a contingency table analysis to determine the association of bronchial dysplasia with treatment (ACAPHA 1 or placebo). All P values are two-sided. A two-sided P value of less than 0.05 was considered to be statistically significant.

To adjust for the effect of various factors on the likelihood of regression of preinvasive lesions, a multiple logistic regression analysis was used. The analysis included the variables: age, sex, and the smoking intensity (pack-years). All analyses were unconditional and tests of statistical significance and confidence intervals (CIs) for odds ratios (ORs) were based on the log-likelihood test.

The characteristics of the participants are shown in Table 50. There was no significant differences in age, sex and smoking history between the ACAPHA 1 and placebo groups. There was also no significant difference in the number of biopsies taken, the proportion of dysplastic lesions or the severity of the dysplastic lesions between the two groups. A total of 421 biopsies were taken in the placebo group (average 5.8 biopsies per subject). Twenty-four of the 421 biopsies (5.7%) were moderate/severe dysplasia and 168 biopsies (39.9%) showed mild dysplasia. In the ACAPHA 1 group, a total of 121 biopsies were taken (average 6.1 per subject). Ten of the 121 biopsies (8.3%) were moderate/severe dysplasia and 36 biopsies (29.8%) showed mild dysplasia.

**Table 50: Characteristics of participants in the Phase IIA Trial of the Effect of ACAPHA 1 on Bronchial Dysplasia**

| **Number of Participants** | | **ACAPHA 1** | **Placebo*** |
|---|---|---|---|
| | | 20 | 73 |
| **Age (Years)** | Median | 58 | 55 |
| | Range | 46-71 | 43-72 |
| **Gender** | Male | 12 | 44 |
| | Female | 8 | 29 |
| **Smoking Status** | Former | 6 | 11 |
| | Current | 14 | 62 |
| **Pack-Years** | Mean | 65 | 46 |
| | Range | 30-85 | 30-72 |

| | | | |
|---|---|---|---|
| * Placebo data are drawn from two concurrent Phase IIb placebo-controlled trials evaluating an identical cohort treated under the identical protocol at the same institution. | | | |

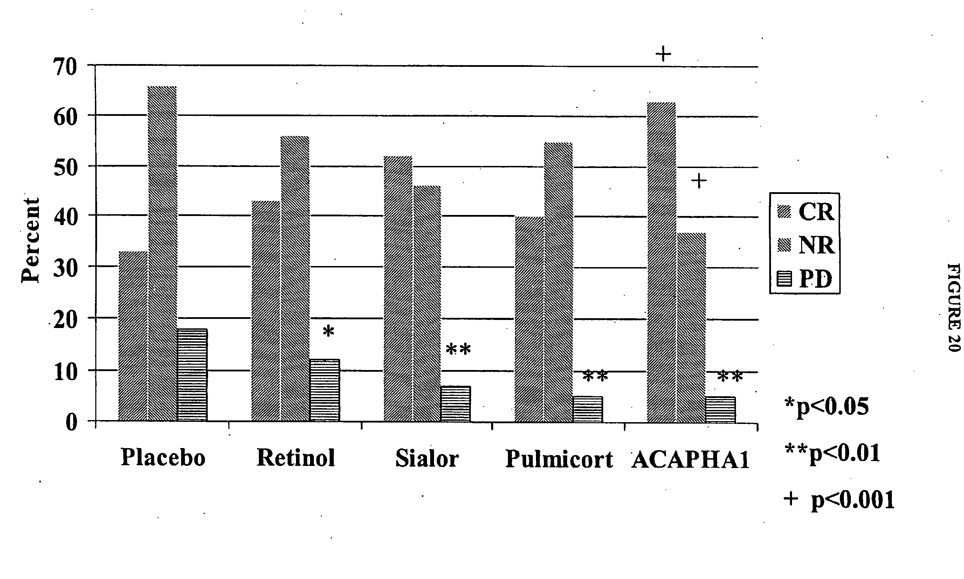

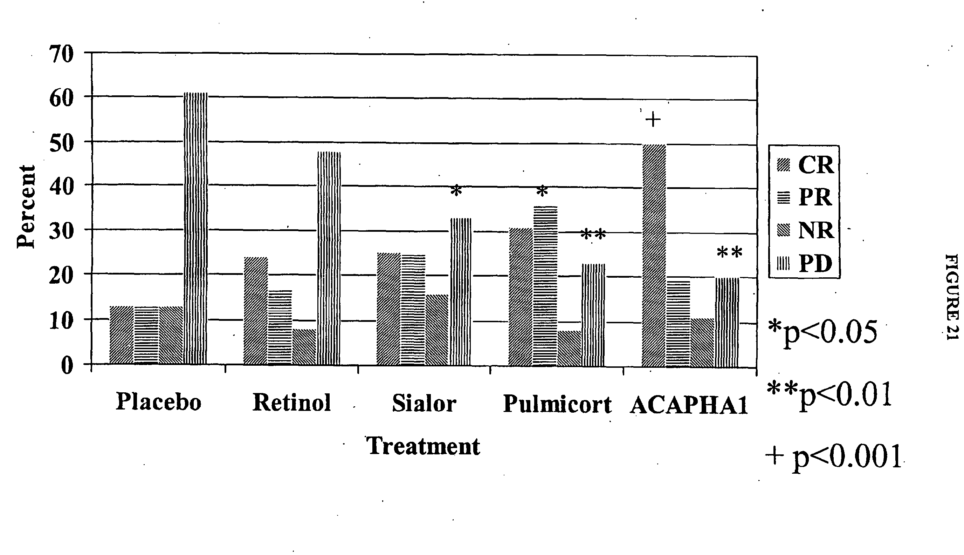

With reference to Figures 20 and 21, at six months, the complete regression rate of areas with dysplasia was significantly higher after taking ACAPHA 1 than placebo (63% versus 33%, P<0.001). The NR and PD rates were also significantly lower (NR 37% versus 66%, P<0.001; PD 5% versus 18%, P=0.008). There were 8 new lesions that were moderate/severe dysplasia and 32 new lesions with mild dysplasia in the placebo group but only 1 new lesion with mild dysplasia in the ACAPHA 1 group. This response is higher than any known chemopreventative agent that has been tested in Phase II/III clinical trials thus far.

In the person specific analysis, the CR rate was 50% in those who took ACAPHA 1 versus 13% in those who were on placebo (p<0.001). The PD rate was also significantly lower in those who were on ACAPHA 1 (20% versus 61 %, P=0.003).

Multiple logistic regression analysis was used to determine the simultaneous effects of age, gender, smoking intensity (pack-years) and the effect of treatment on the CR rate. ACAPHA 1 had a strong effect on the CR rate (P=0.001). Gender had a strong association with CR (P=0.004) suggesting that women had, on average 6.5 times higher odds of CR (95% CI = 1.8 to 23.1 times). None of the remaining variables had a significant association with CR.

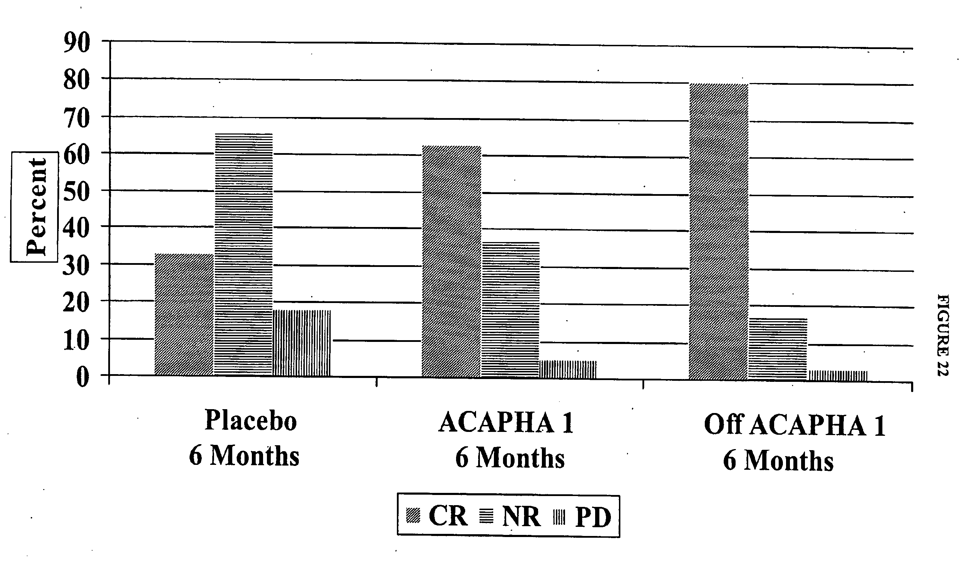

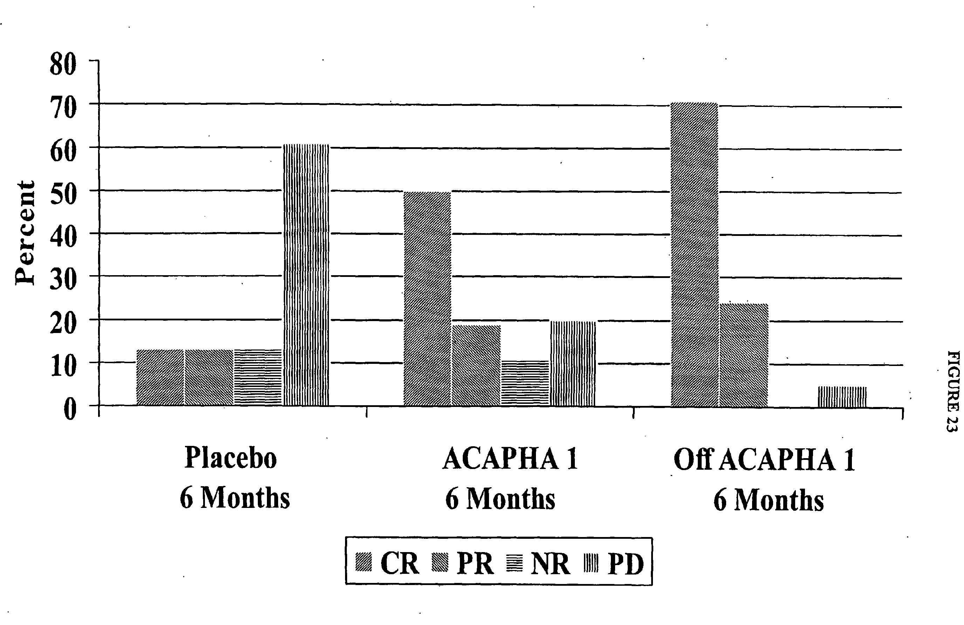

Seventeen subjects were available for the twelve month follow-up assessment after being off ACAPHA for six months. Figure 22 shows, on a site-by-site analysis, the CR, NR and PD rates were 80%, 17% and 3% respectively. Figure 23 shows, on a person specific analysis, the CR, PR and PD rates were 71 %, 24% and 5% respectively. Thus, the effects of ACAPHA 1 were sustained even after the participants were taken off the treatment for six months.

All the participants tolerated the ACAPHA 1 well. There was no symptomatic adverse event. Sub-clinical elevation of AST was observed in 7 of the 20 participants. One of them had pre-existing hepatitis C. Alcohol consumption was thought to be the reason for the AST elevation in another participant. The AST level returned to normal after reducing the dose of ACAPHA 1 to 8 tablets once daily in 4 subjects and discontinuation of the ACAPHA 1 in two subjects. The seventh subject was able to complete the six months course at the full dose with mild elevation of AST only. LD levels were also elevated in a number of patients. The liver enzyme results from some patients are shown below in Table 51. Following these results it is suggested that the ACAPHA 2 or 3 formulation be considered for further application as these formulations are less toxic but are as therapeutically effective.

The results show that in both the subject specific and the lesion specific analyses, there was a significantly better complete response rate in the treatment group than in the control group after 6 months of ACAPHA 1 treatment. There was also a significant reduction in the progression rate in the lesion specific analysis when the ACAPHA 1 treatment group was compared to the placebo group. This response is better than any known chemopreventive agent that has been tested thus far. Figures 21 and 22 compare the efficacy of ACAPHA 1 with placebo, Retinol (Vitamin A), Sialor (Anetholtrithione) and Pulmicort^{™} (Inhaled budesonide). As indicated above, the proportion of subjects having a complete response (CR) was significantly higher than other potential chemopreventive agents. Furthermore, the effects of ACAPHA were sustained even after the participants were taken off the treatment for six months.

**Table 51: ACAPHA 1: Liver Results**

| Month | 1 | 2 | 3 | 3A | 4 | 4A | 48 | 4C | 5 | 5A | 58 | 6 | 6A | 7 | 7A | 7B | 7C | 8 | 9-1YR FIL |
|---|---|---|---|---|---|---|---|---|---|---|---|---|---|---|---|---|---|---|---|
| Patient | | | | | | | | | | | | | | | | | | | |
| 1 | AST-:20 | AST:27 | AST:24 | AST: | AST:23 | AST: | AST: | AST: | AST:28 | AST: | AST: | AST:29 | AST: | AST:+93 | AST:+171 | AST:+295 | AST:+73 | AST: | AST:22 |
| | LD-:103 | LD:122 | LD:127 | LD: | LD:126 | LD: | LD: | LD: | LD:117 | LD: | LD: | LD:120 | LD: | LD:136 | LD: | LD:575 | LD:386 | LD: | LD:109 |
| 2 | AST:27 | AST:26 | AST:27 | AST: | AST:22 | AST: | AST: | AST: | AST:24 | AST: | AST: | AST:20 | AST: | AST:24 | AST: | AST: | AST: | AST: | AST:27 |
| | LD:150 | LD: 149 | LD:168 | LD: | LD:155 | LD: | LD: | LD: | LD:168 | LD: | LD: | LD:157 | LD: | LD:156 | LD: | LD: | LD: | LD: | LD:187 |
| 3 | AST:34 | AST: | AST:+44 | AST: | AST:+58 | AST: | AST: | AST: | AST:+68 | AST:+72 | AST: | AST:+59 | AST: | AST:+73 | AST: | AST: | AST: | AST: | AST:+71 |
| | LD:140 | LD: | LD:175 | LD: | LD:589 | LD: | LD: | LD: | LD:644 | LD:591 | LD: | LD:521 | LD: | LD:194 | LD: | LD: | LD: | LD: | LD:204 |
| 4 | AST:20 | AST:23 | AST:24 | AST: | AST:20 | AST: | AST: | AST: | AST:22 | AST: | AST: | AST:20 | AST: | AST:20 | AST: | AST: | AST: | AST: | AST:22 |
| | LD:123 | LD:128 | LD:128 | LD: | LD:142 | LD: | LD: | LD: | LD:124 | LD: | LD: | LD:123 | LD: | LD:117 | LD: | LD: | LD: | LD: | LD:161 |
| 5 | AST:29 | AST:66 | AST:29 | AST: | AST:27 | AST: | AST: | AST: | AST:32 | AST: | AST: | AST:+146 | AST:+64 | AST:+51 | AST:36 | AST: | AST: | AST: | AST:27 |
| | LD:132 | LD:170 | LD:140 | LD: | LD:145 | LD: | LD: | LD: | LD:176 | LD: | LD: | LD:+212 | LD:533 | LD:167 | LD:157 | LD: | LD: | LD: | LD:165 |
| 6 | AST:20 | AST:+11 | AST:+16 | AST: | AST:+18 | AST: | AST: | AST: | AST:20 | AST: | AST: | AST:19 | AST: | AST:+18 | AST: | AST: | AST: | AST: | AST:22 |
| | LD:369 | LD:138 | LD:138 | LD: | LD:142 | LD: | LD: | LD: | LD:154 | LD: | LD: | LD:131 | LD: | LD:140 | LD: | LD: | LD: | LD: | LD:147 |
| 7 | AST:16 | AST:18 | AST: | AST: | AST:17 | AST: | AST: | AST: | AST:21 | AST: | AST: | AST:20 | AST: | AST:21 | AST: | AST: | AST: | AST: | AST:+18 |
| | LD:147 | LD:174 | LD: | LD: | LD:168 | LD: | LD: | LD: | LD:181 | LD: | LD: | LD:166 | LD: | LD:189 | LD: | LD: | LD: | LD: | LD:192 |
| 8 | AST:28 | AST:30 | AST:33 | AST: | AST:+43 | AST: | AST: | AST: | AST:+191 | AST:+542 | AST:+262 | AST:+68 | AST:+111 | AST:+234 | AST:+220 | AST:+56 | AST:29 | AST: | AST:28 |
| | LD:135 | LD:150 | LD:149 | LD: | LD:157 | LD: | LD: | LD: | LD:+222 | LD:+1060 | LD:+666 | LD:157 | LD:521 | LD:+218 | LD:641 | LD:479 | LD:125 | LD: | LD:139 |
| 9 | AST:20 | AST:21 | AST:31 | AST: | AST:+45 | AST:+45 | AST: | AST: | AST:+46 | AST: | AST: | AST:+60 | AST: | AST:+102 | AST: | AST: | AST: | AST: | AST:24 |
| | LD:103 | LD:117 | LD:127 | LD: | LD:133 | LD:133 | LD: | LD: | LD:124 | LD: | LD: | LD:131 | LD: | LD:161 | LD: | LD: | LD: | LD: | LD:112 |
| 10 | AST:23 | AST:25 | AST:25 | AST: | AST:+301 | AST:+131 | AST:+92 | AST:+73 | AST:+68 | AST: | AST: | AST:+49 | AST: | AST:+70 | AST:+50 | AST: | AST: | AST: | AST:19 |
| | LD:118 | LD:110 | LD:123 | LD: | LD:193 | LD:164 | LD:165 | LD:493 | LD:151 | LD: | LD: | LD:120 | LD: | LD:137 | LD:415 | LD: | LD: | LD: | LD:123 |
| 11 | AST:20 | AST:22 | AST:27 | AST: | AST:29 | AST: | AST: | AST: | AST:32 | AST: | AST: | AST:25 | AST: | AST:25 | AST: | AST: | AST: | AST:22 | AST:33 |
| | LD:97 | LD: 116 | LD:134 | LD: | LD:110 | LD: | LD: | LD: | LD:121 | LD: | LD: | LD: 114 | LD: | LD:117 | LD: | LD: | LD: | LD:113 | LD:118 |
| 12 | AST:22 | AST:19 | AST:+18 | AST: | AST:+ 17 | AST: | AST: | AST: | AST:+17 | AST: | AST: | AST:+18 | AST: | AST:21 | AST: | AST: | AST: | AST: | AST:22 |
| | LD:146 | LD: 168 | LD: 173 | LD: | LD:158 | LD: | LD: | LD: | LD:158 | LD: | LD: | LD: 155 | LD: | LD:176 | LD: | LD: | LD: | LD: | LD:532 |
| 13 | AST:28 | AST:30 | AST:25 | AST: | AST:25 | AST: | AST: | AST: | AST:24 | AST: | AST: | AST:28 | AST: | AST:22 | AST: | AST: | AST: | AST: | AST:27 |
| | LD:161 | LD: 151 | LD: 155 | LD: | LD: 136 | LD: | LD: | LD: | LD:146 | LD: | LD: | LD:147 | LD: | LD: 123 | LD: | LD: | LD: | LD: | LD: 155 |
| 14 | AST:21 | AST:25 | AST:27 | AST: | AST:26 | AST: | AST: | AST: | AST:24 | AST: | AST: | AST:25 | AST: | AST:19 | AST: | AST: | AST: | AST: | AST:30 |
| | LD: 154 | LD: 144 | LD: 154 | LD: | LD: 148 | LD: | LD: | LD: | LD: 138 | LD: | LD: | LD: 167 | LD: | LD:454 | LD: | LD: | LD: | LD: | LD:178 |
| 15 | AST:+17 | AST:+18 | AST:34 | AST: | AST:+15 | AST: | AST: | AST: | AST:19 | AST: | AST: | AST:23 | AST: | AST:+16 | AST: | AST: | AST: | AST: | AST:+17 |
| | LD:130 | LD:147 | LD: 145 | LD: | LD:139 | LD: | LD: | LD: | LD: 154 | LD: | LD: | LD:127 | LD: | LD: 102 | LD: | LD: | LD: | LD: | LD:148 |
| 16 | AST:30 | AST:30 | AST:30 | AST: | AST:31 | AST: | AST: | AST: | AST:31 | AST: | AST: | AST:26 | AST: | AST:29 | AST: | AST: | AST: | AST: | AST:31 |
| | LD:163 | LD:189 | LD:189 | LD: | LD: 158 | LD: | LD: | LD: | LD:158 | LD: | LD: | LD: 150 | LD: | LD:162 | LD: | LD: | LD: | LD: | LD: 166 |
| 17 | AST:19 | AST:21 | AST: | AST: | AST:18 | AST: | AST: | AST: | AST:+15 | AST: | AST: | AST:19 | AST: | AST:17 | AST: | AST: | AST: | AST: | AST:20 |
| | LD: 113 | LD:132 | LD: | LD: | LD:124 | LD: | LD: | LD: | LD:130 | LD: | LD: | LD:129 | LD: | LD:401 | LD: | LD: | LD: | LD: | LD:413 |
| 18 | AST:24 | AST:25 | AST:26 | AST: | AST:27 | AST: | AST: | AST: | AST:27 | AST: | AST: | AST:20 | AST: | AST:27 | AST: | AST: | AST: | AST: | AST;31 |
| | LD:137 | LD: 140 | LD:147 | LD: | LD:147 | LD: | LD: | LD: | LD: 130 | LD: | LD: | LD:132 | LD: | LD: 154 | LD: | LD: | LD: | LD: | LD:153 |
| 19 | AST:21 | AST:+16 | AST:+16 | AST: | AST:+17 | AST: | AST: | AST: | AST:+16 | AST: | AST: | AST:+15 | AST: | AST:20 | AST: | AST: | AST: | AST: | AST: |
| | LD: 113 | LD:120 | LD:129 | LD: | LD: 125 | LD: | LD: | LD: | LD:143 | LD: | LD: | LD:133 | LD: | LD:125 | LD: | LD: | LD: | LD: | LD: |
| 20 | AST:+43 | AST: | AST:+161 | AST:+113 | AST:+65 | AST: | AST: | AST: | AST:+179 | AST:+323 | AST: | AST:+83 | AST:+50 | AST:32 | AST: | AST: | AST: | AST: | AST:20 |
| | LD:141 | LD:146 | LD:146 | LD:478 | LD:132 | LD: | LD: | LD: | LD:209 | LD:+687 | LD: | LD:136 | LD:361 | LD:139 | LD: | LD: | LD: | LD: | LD:129 |
| 21 | AST:+43 | AST: | AST:+161 | AST:+ 113 | AST:+65 | AST: | AST: | AST: | AST:+179 | AST:+323 | AST: | AST:+83 | AST:+50 | AST:32 | AST: | AST: | AST: | AST: | AST:20 |
| | LD:141 | LD:146 | LD:146 | LD:478 | LD:132 | LD: | LD: | LD: | LD:209 | LD:+687 | LD: | LD:136 | LD:361 | LD:139 | LD: | LD: | LD: | LD: | LD:129 |

| | | | | | | | | | | | | | | | | | | | |
|---|---|---|---|---|---|---|---|---|---|---|---|---|---|---|---|---|---|---|---|
| *AST(Aspartate Aminotransferase) = U/L **LD (Lactic Dehydrogenase) = U/L | | | | | | | | | | | | | | | | | | | |

## Claims

1. A use of an effective amount of a composition comprising a mixture of *Sophora tonkinensis, Polygonum bistorta, Prunella vulgaris , Sonchus brachyotus, Dictamnus dasycarpus* and *Dioscorea bulbifera* for the production of a medicament for preventing or treating cancer in a patient in need thereof; wherein the cancer is selected from the group consisting of lung cancer, prostate cancer, bladder cancer, breast cancer and leukaemia, wherein the first five herbs are present in an amount from 5% to 33% and the sixth herb is present in an amount from 1.0% to 1.4% or wherein the first four herbs are present in an amount from 5% to 38% and the fifth herb is present in an amount from 2.5% to 19% and the sixth herb is present in an amount from 1.0% to 1.4%.

2. A use of an effective amount of a composition comprising a mixture of *Sophora tonkinensis, Polygonum bistorta* and *Prunella vulgaris, Sonchus brachyotus, Dictamnus dasycarpus* and *Dioscorea bulbifera* for the production of a medicament for treating *Helicobacter pylori* infection in a patient in need thereof.

3. A use of an effective amount of a composition comprising a mixture of *Sophora tonkinensis, Polygonum bistorta, Prunella vulgaris, Sonchus brachyotus, Dictamnus dasycarpus* and *Dioscorea bulbifera* for the production of a medicament for treating or reducing the reoccurrence of cancer in a patient in need, wherein the first five herbs are present in an amount from 5% to 33% and the sixth herb is present in an amount from 1.0% to 1.4% or wherein the first four herbs are present in an amount from 5% to 38% and the fifth herb is present in an amount from 2.5% to 19% and the sixth herb is present in an amount from 1.0% to 1.4%.

4. The use of claim 3, wherein said composition is used as an adjunct to conventional surgery, chemotherapy or radiation therapy treatments.

5. A use of an effective amount of a composition comprising a mixture of *Sophora tonkinensis, Polygonum bistorta* and *Prunella vulgaris, Sonchus brachyotus, Dictamnus dasycarpus* and *Dioscorea buibifera* for the production of a medicament for treating or preventing a chronic inflammatory condition in a patient in need thereof.

6. A use of an effective amount of a composition comprising a mixture of *Sophora tonkinensis, Polygonum bistorta* and *Prunella vulgaris, Sonchus brachyotus, Dictamnus dasycarpus* and *Dioscorea bulbifera* for the production of a medicament for treating or preventing cardiovascular disease in a patient in need thereof.

7. A use of an effective amount of a composition comprising a mixture of *Sophora tonkinensis, Polygonum bistorta* and *Prunella vulgaris, Sonchus brachyotus, Dictamnus dasycarpus* and *Dioscorea bulbifera* for the production of a medicament for treating or preventing cerebral vascular disease in a patient in need thereof.

8. A use of an effective amount of a composition comprising a mixture of *Sophora tonkinensis, Polygonum bistorta , Prunella vulgaris, Sonchus brachyotus, Dictamnus dasycarpus* and *Dioscorea bulbifera* for the production of a medicament for preventing the occurrance of or treating precancerous cells in a patient in need thereof, wherein the first five herbs are present in an amount from 5% to 33% and the sixth herb is present in an amount from 1.0% to 1.4% or wherein the first four herbs are present in an amount from 5% to 38% and the fifth herb is present in an amount from 2.5% to 19% and the sixth herb is present in an amount from 1.0% to 1.4%.

9. The use of Claim 8, wherein the composition is used for the prevention or treatment of bronchial dysplasia.

10. The use of Claim 9, wherein the composition is used for the prevention or treatment of bronchial dysplasia wherein the method is for the prevention or treatment of chronic obstructive pulmonary disease.

11. A use of an effective amount of a composition comprising a mixture of *Sophora tonkinensis, Polygonum bistorta* and *Prunella vulgaris, Sonchus brachyotus, Dictamnus dasycarpus* and *Dioscorea bulbifera* for the production of a medicament for treating or preventing atrophic gastritis in a patient in need thereof.

12. A use of an effective amount of a composition comprising a mixture of *Sophora tonkinensis, Polygonum bistorta* and *Prunella vulgaris, Sonchus brachyotus, Dictamnus dasycarpus* and *Dioscorea bulbifera* for the production of a medicament for reducing adverse effects of radiation therapy.

13. A composition consisting of (i) a mixture of six herbs including 5% to 33% *Sophora tonkinensis,* 5% to 33% *Polygonum bistorta ,* 5% to 33% *Prunella vulgaris,* 5% to 33% *Sonchus brachyotus,* 5% to 33% *Dictamnus dasycarpus* and 1.0% to 1.4% *Dioscorea* bulbifera or (ii) a mixture of six herbs including 5% to 38% *Sophora tonkinensis,* 5% to 38% *Polygonum bistorta ,* 5% to 38% *Prunella vulgaris,* 5% to *38% Sonchus brachyotus,* 2.5% to 19% *Dictamnus dasycarpus* and 1.0 to 1.4% *Dioscorea bulbifera.*

## Patentansprüche

1. Benutzung einer wirksamen Menge einer Zusammensetzung, umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Verhüten oder zum Behandeln von Krebs in einem bedürftigen Patienten; wobei der Krebs aus der Gruppe bestehend aus Lungenkrebs, Prostatakrebs, Blasenkrebs, Brustkrebs und Leukämie ausgewählt wird, wobei die ersten fünf Kräuter in einer Menge von 5% bis 33% vorhanden sind und das sechste Kraut in einer Menge von 1,0% bis 1,4% vorhanden ist oder wobei die ersten vier Kräuter in einer Menge von 5% bis 38% vorhanden sind und das fünfte Kraut in einer Menge von 2,5% bis 19% vorhanden ist und das sechste Kraut in einer Menge von 1,0% bis 1,4% vorhanden ist.

2. Benutzung einer wirksamen Menge einer Zusammensetzung umfassend eine Mischung von *Sophora tonkinensis, Polygonum bistorta* und *Prunella vulgaris, Sonchus brachyotus, Dictamnus dasycarpus* und *Dioscorea bulbifera* für die Herstellung eines Medikamentes zum Behandeln einer *Helicobacter pylori* Infektion in einem bedürftigen Patienten.

3. Benutzung einer wirksamen Menge einer Zusammensetzung, umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Behandeln oder Verringern des Wiederauftretens von Krebs in einem bedürftigen Patienten, wobei die ersten fünf Kräuter in einer Menge von 5% bis 33% vorhanden sind und das sechste Kraut in einer Menge von 1,0% bis 1,4% vorhanden ist oder wobei die ersten vier Kräuter in einer Menge von 5% bis 38% vorhanden sind und das fünfte Kraut in einer Menge von 2,5% bis 19% vorhanden ist und das sechste Kraut in einer Menge von 1,0% bis 1,4% vorhanden ist.

4. Benutzung nach Anspruch 3, wobei die Zusammensetzung als eine Beigabe bei konventionellen Chirurgie, Chemotherapie oder Strahlungstherapie-Behandlungen benutzt wird.

5. Benutzung einer wirksamen Menge einer Zusammensetzung umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Behandeln oder Verhüten eines chronischen Entzündungszustandes in einem bedürftigen Patienten.

6. Benutzung einer wirksamen Menge einer Zusammensetzung umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Behandeln oder Verhüten von kardiovaskulärer Krankheit in einem bedürftigen Patienten.

7. Benutzung einer wirksamen Menge einer Zusammensetzung umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Behandeln oder Verhüten von gehirnvaskulärer Krankheit in einem bedürftigen Patienten.

8. Benutzung einer wirksamen Menge einer Zusammensetzung umfassend eine Mischung von *Sophora tonkinensis, Polygonum bistorta, Prunella vulgaris, Sonchus brachyotus Dictamnus dasycarpus* und *Dioscorea bulbifera* für die Herstellung eines Medikamentes zum Verhüten des Auftretens von oder zum Behandeln von vorkrebsbefallenen Zellen in einem bedürftigen Patienten, wobei die ersten fünf Kräuter in einer Menge von 5% bis 33% vorhanden sind und das sechste Kraut in einer Menge von 1,0% bis 1,4% vorhanden ist oder wobei die ersten vier Kräuter in einer Menge von 5% bis 38% vorhanden sind und das fünfte Kraut in einer Menge von 2,5% bis 19% vorhanden ist und das sechste Kraut in einer Menge von 1,0% bis 1,4% vorhanden ist.

9. Benutzung nach Anspruch 8, wobei die Zusammensetzung für die Verhütung oder Behandlung von bronchialer Dysplasie benutzt wird.

10. Benutzung nach Anspruch 9, wobei die Zusammensetzung für die Verhütung oder Behandlung von bronchialer Dysplasie benutzt wird, wobei das Verfahren für die Verhütung oder Behandlung von chronischer, versperrender Lungenkrankheit ist.

11. Benutzung einer wirksamen Menge einer Zusammensetzung umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Behandeln oder Verhüten von atrophischer Gastritis in einem bedürftigen Patienten.

12. Benutzung einer wirksamen Menge einer Zusammensetzung umfassend eine Mischung von *Sophora tonkinensis, Polygonum bistorta, Prunella vulgaris, Sonchus brachyotus, Dictamnus dasycarpus* und *Dioscorea bulbifera* für die Herstellung eines Medikamentes zum Verringern von negativen Auswirkungen einer Strahlungstherapie.

13. Zusammensetzung vorwiegend bestehend aus (i) einer Mischung von sechs Kräutern einschliesslich 5% bis 33% *Sophora tonkinensis,* 5% bis 33% *Polygonum bistoria,* 5% bis 33% *Prunella vulgaris"* 5% bis 33% *Sonchus brachyotus,* 5% bis 33% *Dictamnus dasycarpus* und 1,0% bis 1,4% *Dioscorea bulbifera* oder (ii) einer Mischung von sechs Kräutern einschliesslich 5% bis 38% *Sophora tonkinensis,* 5% bis 38% *Polygonum bistorta*, 5% bis 38% *Prunella vulgaris,* 5% bis 38% *Sonchus brachyotus,* 2,5% bis 19% *Dictamnus dasycarpus* und 1,0% bis 1,4% *Dioscorea bulbifera.*

## Revendications

1. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera pour la production d'un médicament destiné à prévenir ou à traiter un cancer chez un patient, ce cancer figurant dans le groupe constitué du cancer du poumon, du cancer de la prostate, du cancer de la vessie, du cancer du sein et de la leucémie, où les cinq premières herbes sont présentes dans une proportion de 5 % à 33 % et la sixième herbe est présente dans une proportion de 1,0 % à 1,4 % ou les quatre premières herbes sont présentes dans une proportion de 5 % à 38 %, la cinquième herbe est présente dans une proportion de 2,5 % à 19 % et la sixième herbe est présente dans une proportion de 1,0 % à 1,4%.

2. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera pour la production d'un médicament destiné à traiter l'infection à Helicobacter pylori chez un patient.

3. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera pour la production d'un médicament destiné à traiter ou à réduire le risque de réapparition d'un cancer chez un patient, où les cinq premières herbes sont présentes dans une proportion de 5 % à 33 % et la sixième herbe est présente dans une proportion de 1,0 % à 1,4 % ou les quatre premières herbes sont présentes dans une proportion de 5 % à 38 %, la cinquième herbe est présente dans une proportion de 2,5 % à 19 % et la sixième herbe est présente dans une proportion de 1,0 % à 1,4 %.

4. Utilisation selon la revendication 3, dans laquelle la composition est utilisée en tant que traitement d'appoint à des traitements de chirurgie, chimiothérapie ou radiothérapie conventionnels.

5. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera pour la production d'un médicament destiné à traiter ou à prévenir une pathologie inflammatoire chronique chez un patient.

6. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera, pour la production d'un médicament destiné à traiter ou à prévenir une maladie cardiovasculaire chez un patient.

7. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera, pour la production d'un médicament destiné à traiter ou à prévenir une maladie vasculaire cérébrale chez un patient.

8. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera pour la production d'un médicament destiné à prévenir l'apparition de cellules précancéreuses ou à traiter des cellules précancéreuses chez un patient, où les cinq premières herbes sont présentes dans une proportion de 5 % à 33 % et la sixième herbe est présente dans une proportion de 1,0 % à 1,4 %, ou les quatre premières herbes sont présentes dans une proportion de 5 % à 38 %, la cinquième herbe est présente dans une proportion de 2,5 % à 19 % et la sixième herbe est présente dans une proportion de 1,0 % à 1,4 %.

9. Utilisation selon la revendication 8, dans laquelle la composition est utilisée pour la prévention ou le traitement de la dysplasie bronchique.

10. Utilisation selon la revendication 9, dans laquelle la composition est utilisée pour la prévention ou le traitement de la dysplasie bronchique, le procédé ayant pour but la prévention ou le traitement d'une maladie pulmonaire obstructive chronique.

11. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera, pour la production d'un médicament destiné à traiter ou à prévenir la gastrite atrophique chez un patient.

12. Utilisation d'une quantité efficace d'une composition comprenant un mélange de Sophora tonkinensis, Polygonum bistorta, Prunella vulgaris, Sonchus brachyotus, Dictamnus dasycarpus et Dioscorea bulbifera, pour la production d'un médicament destiné à réduire les effets indésirables de la radiothérapie.

13. Composition constituée de (i) un mélange de six herbes comprenant 5 % à 33 % de Sophora tonkinensis, 5 % à 33 % de Polygonum bistorta, 5 % à 33 % de Prunella vulgaris, 5 % à 33 % de Sonchus brachyotus, 5 % à 33 % de Dictamnus dasycarpus et 1,0 % à 1,4 % de Dioscorea bulbifera ou (ii) un mélange de six herbes comprenant 5 % à 38 % de Sophora tonkinensis, 5 % à 38 % de Polygonum bistorta, 5 % à 38 % de Prunella vulgaris, 5 % à 38 % de Sonchus brachyotus, 2,5 % à 19 % de Dictamnus dasycarpus et 1,0 % à 1,4 % de Dioscorea bulbifera.
